# EUROPEAN PATENT SPECIFICATION

(11) **EP 4 108 167 B1**
(45) Date of publication and mention of the grant of the patent: **21.01.2026**
(21) Application number: 20919740.9
(22) Date of filing: 18.11.2020
(51) Int. Cl.: A61B 5/0295, A61B 5/00, A61B 5/024

(54) **METHOD AND DEVICE FOR GENERATING PHOTOPLETHYSMOGRAPHY SIGNAL**
VERFAHREN UND VORRICHTUNG ZUR ERZEUGUNG FOTOPLETHYSMOGRAFISCHER SIGNALE
PROCÉDÉ ET DISPOSITIF POUR GÉNÉRER UN SIGNAL DE PHOTOPLÉTHYSMOGRAPHIE

(30) Priority: 21.02.2020 CN 202010110278
(43) Date of publication of application: 28.12.2022
(73) Proprietor: Lepu Medical Technology (Beijing) Co., Ltd., Beijing 102200 (CN)
(72) Inventor: WU, Zejian, Beijing 102200 (CN); WANG, Bin, Beijing 102200 (CN); WANG, Sihan, Beijing 102200 (CN); CAO, Jun, Beijing 102200 (CN)
(74) Representative: Glück Kritzenberger Patentanwälte PartGmbB
(86) International application number: PCT/CN2020/129640
(87) International publication number: WO 2021/164350

(56) References cited:
- EP-A1- 3 207 862
- CN-A- 104 639 799
- CN-A- 109 820 499
- CN-A- 111 297 347
- US-A1- 2016 302 674
- US-A1- 2017 007 185
- US-A1- 2018 214 088
- US-A1- 2018 360 330
- US-B2- 10 398 353

## Description

The application claims priority to Chinese Patent Application No. 202010110278.X, entitled "Method and Device for Generating Photoplethysmography(PPG) Signals", filed with the China National Intellectual Property Administration on February 21, 2020.

### BACKGROUND OF THE INVENTION

### 1. Technical Field

The invention relates to the technical field of electrophysiological signal processing, in particular to a method and device for generating PPG signals.

### 2. Description of Related Art

The work condition of the heart is affected by many factors such as the current health, hormone level, emotional state and even life style of people, and conversely, the health and disease information of people can be obtained by analyzing the motion trajectory of the heart. To obtain motion trajectory data of the heart, it is necessary to monitor heart motions and acquire heart data, which are traditionally completed through an electrocardiogram (ECG) monitoring technique. By adoption of this technique, lead electrodes are connected to multiple parts of the human body to acquire ECG signals. During specific operation, a monitored object has to lie still or wear multiple contact devices (heart patches, heart belts, or the like) all the time in daily life, which causes much inconvenience to the activities and normal life of the monitored object.

Photoplethysmography (PPG) is a non-invasive detection method for detecting the change of blood volume in viable tissues through a photoelectric method. When the heart beats, the blood flow in unit area in blood vessels changes periodically, the blood volume changes correspondingly, and a PPG signal reflecting the light absorption capacity of blood also changes periodically. The PPG signal may be acquired by measuring the fingers, ears or other parts. A series of oriented analysis of heart activities may be carried out through the PPG signals, and compared with the ECG method, the PPG method may improve the comfort of the monitored object. An example of such a method is for example EP3207862 . However, in actual application, we find that existing PPG signal acquisition devices and methods are inflexible: only a red light source or an infrared light source may be used as a radiation source, an acquisition front end is generally a customized mechanical device such as a finger clip or an ear clip, and acquired data have to be analyzed through specific sensors. All these problems make it more difficult to acquire PPG signal data through an upper medical application.

### BRIEF SUMMARY OF THE INVENTION

The objective of the invention is to overcome the defects of the prior art by providing a method and device for generating PPG signals. According to the method and device, a mobile terminal is used to photograph the skin surface, so that data is acquired independent of a customized device; a video is recoded and is analyzed to generate a PPG signal, so that the data generation process no longer depends on specific sensor analysis modules; and remote backup of the recoded video and data analysis and processing of the remote video are provided, so that the data sharing degree of an upper application is improved, and the upper application can establish a large PPG database.

To fulfill the above objective, in a first aspect, the embodiments of the invention provide a method for generating PPG signals, comprising:
Performing a continuous acquisition and photographing operation on a local skin surface of a living body by a mobile terminal, and in the continuous acquisition and photographing process, performing continuous video fragment caching on video data obtained by photographing with a preset cached fragment time threshold as a fragment length to generate multiple cached fragment video data;
Performing frame image extraction on the cached fragment video data to generate a cached fragment frame image sequence, and performing image quality detection on all cached fragment frame images in the image sequence of cached fragment frame according to a preset red light pixel threshold range to generate an image quality detection result, wherein the image sequence of cached fragment frame comprises multiple cached fragment frame images;
When the image quality detection result is an up-to-standard image identifier, performing one-dimensional red light signal extraction on all the cached fragment frame images in the image sequence of cached fragment frame according to the red light pixel threshold range to generate a first red light digital signal, and performing one-dimensional green light signal extraction on all the cached fragment frame images in the image sequence of cached fragment frame according to a preset green light pixel threshold range to generate a first green light digital signal; and
Performing signal band-pass filtering preprocessing on the first red light digital signal according to a preset band-pass filtering frequency threshold range to generate a second red light digital signal, and performing signal band-pass filtering preprocessing on the first green light digital signal according to the preset band-pass filtering frequency threshold range to generate a second green light digital signal; performing maximum frequency difference determination on the second red light digital signal and the second green light digital signal to generate a first determination result; performing signal-to-noise ratio determination on the second red light digital signal and the second green light digital signal to generate a second determination result when the first determination result is an up-to-standard signal identifier; and performing PPG signal generation on the second red light digital signal and the second green light digital signal to generate a first PPG signal of the cached fragment video data when the second determination result is the up-to-standard signal identifier.

Preferably, the method further comprises:
Performing graphic transform on the first PPG signal by the mobile terminal to generate a PPG waveform image of a current fragment, and locally displaying the PPG waveform image of the current fragment in real time through a display interface.

Preferably, the method further comprises:
After the continuous acquisition and photographing operation, performing video stitching on all the cached fragment video data in chronological order by the mobile terminal to generate complete skin surface video data, and sending the video data of complete skin surface to a remote server;
Performing frame image extraction on the video data of complete skin surface by the server to generate a complete skin surface video frame image sequence, wherein the complete skin surface video frame image sequence comprises multiple complete skin surface video frame images;
Performing one-dimensional red light signal extraction on all the complete skin surface video frame images in the complete skin surface video frame image sequence according to a preset server red light pixel threshold range to generate a first server red light digital signal, and performing one-dimensional green light signal extraction on all the complete skin surface video frame images in the complete skin surface video frame image sequence according to a preset server green light pixel threshold range to generate a first server green light digital signal;
According to a preset server band-pass filtering frequency threshold range, performing signal band-pass filtering preprocessing on the first server red light digital signal to generate a second server red light digital signal, and performing signal band-pass filtering preprocessing on the first server green light digital signal to generate a second server green light digital signal; performing maximum frequency difference determination on the second server red light digital signal and the second server green light digital signal to generate a first server determination result; when the first server determination result is the up-to-standard signal identifier, performing signal-to-noise ratio determination on the second server red light digital signal and the second server green light digital signal to generate a second server determination result; and when the second server determination result is the up-to-standard signal identifier, performing PPG signal generation on the second server red light digital signal and the second server green light digital signal to generate a second PPG signal of the complete skin surface video data; and
Performing PPG data file conversion on the second PPG signal by the server to generate a complete skin surface PPG data file, and saving the video data of complete skin surfaceand the complete skin surface PPG data file in a medical database.

Preferably, the step of performing frame image extraction on the cached fragment video data to generate a cached fragment frame image sequence, and performing image quality detection on all cached fragment frame images in the image sequence of cached fragment frame according to a preset red light pixel threshold range to generate an image quality detection result specifically comprises:
Sequentially performing data fragment extraction on the cached fragment video data according to a preset cached fragment frame data length to obtain multiple data fragments, generating the multiple cached fragment frame images according to the multiple data fragments, and sorting all the cached fragment frame images in order to generate the image sequence of cached fragment frame ; and
Sequentially performing cached fragment frame image extraction on the image sequence of cached fragment frame in chronological order to generate a current cached fragment frame image; calculating a total number of pixels in the current cached fragment frame image to generate a total pixel number, and calculating a total number of pixels with a pixel value within the red light pixel threshold range to generate a total red light pixel number; generating a red proportion parameter of a current frame according to a ratio of the total red light pixel number to the total pixel number; performing image quality detection on the red proportion parameter of the current frame according to a preset red proportion lower threshold; when the red proportion parameter of the current frame is less than the red proportion lower threshold, setting the image quality detection result as a not-up-to-standard image identifier, and stopping cached fragment frame image extraction performed on the image sequence of cached fragment frame ; or, when the red proportion parameter of the current frame is greater than or equal to the red proportion lower threshold, setting the image quality detection result as the up-to-standard image identifier, and continuing to perform cached fragment frame image extraction on the image sequence of cached fragment frame .

The step of when the image quality detection result is an up-to-standard image identifier, performing one-dimensional red light signal extraction on all the cached fragment frame images in the image sequence of cached fragment frame according to the red light pixel threshold range to generate a first red light digital signal, and performing one-dimensional green light signal extraction on all the cached fragment frame images in the image sequence of cached fragment frame according to a preset green light pixel threshold range to generate a first green light digital signal specifically comprises:
Step 51, when the image quality detection result is the up-to-standard image identifier, initializing the first red light digital signal to be null, initializing the first green light digital signal to be null, initializing a first index to 1, and initializing a first total number to a total number of the cached fragment frame images in the image sequence of cached fragment frame ;
Step 52, setting a first-index frame image as the cached fragment frame image, corresponding to the first index, in the image sequence of cached fragment frame ;
Step 53, collecting all pixels, meeting the red light pixel threshold range, in the first-index frame image to generate a red pixel set, calculating a total number of pixels included in the red pixel set to generate a red pixel total number, calculating the sum of pixel values of all the pixels in the red pixel set to generate a red pixel value sum, and generating first-index frame red light channel data according to a quotient obtained by dividing the red pixel value sum by the red pixel total number; and adding the first-index frame red light channel data into the first red light digital signal as signal point data;
Step 54, collecting all pixels, meeting the green light pixel threshold range, in the first-index frame image to generate a green pixel set, calculating a total number of pixels included in the green pixel set to generate a green pixel total number, calculating the sum of pixel values of all the pixels in the green pixel set to generate a green pixel value sum, and generating first-index frame green light channel data according to a quotient obtained by dividing the green pixel value sum by the green pixel total number; and adding the first-index frame green light channel data into the first green light digital signal as signal point data;
Step 55, increasing the first index by 1;
Step 56, determining whether the first index is greater than the first total number; if the first index is less than or equal to the first total number, performing Step 52; or, if the first index is greater than the first total number, performing Step 57; and
Step 57, transferring the first red light digital signal to an upper processing process as a one-dimensional red light signal extraction result, and transferring the first green light digital signal to the upper processing process as a one-dimensional green light signal extraction result.

The step of performing signal band-pass filtering preprocessing on the first red light digital signal according to a preset band-pass filtering frequency threshold range to generate a second red light digital signal, and performing signal band-pass filtering preprocessing on the first green light digital signal according to the preset band-pass filtering frequency threshold range to generate a second green light digital signal specifically comprises:
According to the band-pass filtering frequency threshold range, performing digital signal filtering processing on low-frequency noise signal points with a signal frequency lower than the band-pass filtering frequency threshold range and high-frequency noise signal points with a signal frequency higher than the band-pass filtering frequency threshold range in the first red light digital signal to generate the second red light digital signal; and
According to the band-pass filtering frequency threshold range, performing digital signal filtering processing on low-frequency noise signal points with a signal frequency lower than the band-pass filtering frequency threshold range and high-frequency noise signal points with a signal frequency higher than the band-pass filtering frequency threshold range in the first green light digital signal to generate the second green light digital signal.

Preferably, the step of performing maximum frequency difference determination on the second red light digital signal and the second green light digital signal to generate a first determination result, performing signal-to-noise ratio determination on the second red light digital signal and the second green light digital signal to generate a second determination result when the first determination result is an up-to-standard signal identifier, and performing PPG signal generation on the second red light digital signal and the second green light digital signal to generate a first PPG signal of the cached fragment video data when the second determination result is the up-to-standard signal identifier specifically comprises:
Step 71, performing digital signal time domain-frequency domain conversion on the second red light digital signal through discrete Fourier transform to generate a red light frequency domain signal, performing digital signal time domain-frequency domain conversion on the second green light digital signal through discrete Fourier transform to generate a green light frequency domain signal; extracting a maximum-energy frequency from the red light frequency domain signal to generate a maximum red light frequency, and extracting a maximum-energy frequency from the green light frequency domain signal to generate a maximum green light frequency; calculating a frequency difference between the maximum red light frequency and the maximum green light frequency to generate a maximum red-green frequency difference; when the maximum red-green frequency difference does not exceed a preset maximum frequency difference threshold range, setting the first determination result as the up-to-standard signal identifier; or, when the maximum red-green frequency difference exceeds the maximum frequency difference threshold range, setting the first determination result as the not-up-to-standard signal identifier;
Step 72, when the first determination result is the up-to-standard signal identifier, according to a preset band-stop filtering frequency threshold range, removing valid signal points with a signal frequency meeting the band-stop filtering frequency threshold range from the second red light digital signal through multi-order Butterworth band-stop filtering to generate a red light noise signal, and removing valid signal points with a signal frequency meeting the band-stop filtering frequency threshold range from the second green light digital signal through multi-order Butterworth band-stop filtering to generate a green light noise signal;
Step 73, calculating signal energy of the second red light digital signal to generate red light signal energy, calculating signal energy of the red light noise signal to generate red light noise energy, generating valid red light signal energy according to a difference between the red light signal energy and the red light noise energy, and generating a red light signal-to-noise ratio according to a ratio of the valid red light signal energy to the red light noise energy;
Step 74, calculating signal energy of the second green light digital signal to generate green light signal energy, calculating signal energy of the green light noise signal to generate green light noise energy, generating valid green light signal energy according to a difference between the green light signal energy and the green light noise energy, and generating a green light signal-to-noise ratio according to a ratio of the valid green light signal energy to the green light noise energy;
Step 75, when the red light signal-to-noise ratio and the green light signal-to-noise ratio are both less than a preset signal-to-noise threshold, setting the second determination result as the not-up-to-standard signal identifier; or, when any one of the red light signal-to-noise ratio and the green light signal-to-noise ratio is greater than or equal to the signal-to-noise threshold, setting the second determination result as the up-to-standard signal identifier;
Step 76, when the second determination result is the up-to-standard signal identifier, setting a red light digital signal of the first PPG signal as the second red light digital signal, and setting a green light digital signal of the first PPG signal as the second green light digital signal, wherein the first PPG signal comprises the red light digital signal and the green light digital signal.

Preferably, the method further comprises:
When the image quality detection result is the not-up-to-standard signal identifier, stopping the continuous acquisition and photographing operation and generating out-of-skin surface error information, then transferring the out-of-skin surface error information to the mobile terminal, and displaying, by the mobile terminal, the out-of-skin surface error information as warning information through the display interface;
When the first determination result is the not-up-to-standard signal identifier, stopping the continuous acquisition and photographing operation and the PPG signal generation process and generating signal quality error information, then transferring the signal quality error information to the mobile terminal, and displaying, by the mobile terminal, the signal quality error information as warning information through the display interface; or
When the second determination result is the not-up-to-standard signal identifier, stopping the continuous acquisition and photographing operation and the PPG signal generation process and generating signal quality error information, then transferring the signal quality error information to the mobile terminal, and displaying, by the mobile terminal, the signal quality error information as warning information through the display interface.

According to the method for generating PPG signals provided by the embodiments of the invention in the first aspect, a customized acquisition device is not needed, and the camera of a common mobile terminal can be used to photograph the skin surface; a valid PPG signal is generated by extracting pixel values of a video and performing denoising and feature extraction on extracted signals through a conventional signal processing method such as band-passing filtering, frequency difference comparison and band-stop filtering rather than analyzing video data obtained by photographing with a customized sensor analysis module; and remote backup of the recoded video and remote data analysis are provided to realize PPG signal generation, so that the data sharing degree of an upper application is improved, and the upper application can establish a large PPG database.

In a second aspect, the embodiments of the invention provide equipment, comprising a memory and a processor, wherein the memory is used to store a program, and the processor is used to implement the method in the first aspect and in all implementations of the first aspect.

In a third aspect, the embodiments of the invention provide a computer program product comprising instructions, wherein the computer program product enables a computer to implement the method in the first aspect and in all implementations of the first aspect when running on the computer.

In a fourth aspect, the embodiments of the invention provide a computer-readable storage medium having a computer program stored therein, wherein when the computer program is executed by a processor, the method in the first aspect and in all implementations of the first aspect is implemented.

### BRIEF DESCRIPTION OF THE SEVERAL VIEWS OF THE DRAWINGS

FIG. 1 is a schematic diagram of a method for generating PPG signals according to Embodiment 1 of the invention;
FIG. 2 is a schematic diagram of a method for generating PPG signals according to Embodiment 2 of the invention;
FIG. 3 is a structural diagram of a device for generating PPG signals according to Embodiment 3 of the invention.

### DETAILED DESCRIPTION OF THE INVENTION

To gain a better understanding of the purposes, technical solutions and advantages of the invention, the invention will be described in further detail below in conjunction with the accompanying drawings. Clearly, the embodiments in the following description are merely illustrative ones, and are not all possible ones of the invention. The scope of the invention is defined in the claims.

Before the invention is expounded in further detail with reference to embodiments, some technical means involved in this specification will be briefly introduced below.

Conventionally, a PPG signal is acquired as follows: the skin surface is irradiated with a specific light source, and then a light signal reflected or transmitted by the skin surface is received by a specific light receiving sensor; and an analysis module of the sensor records and analyzes light intensity changes in chronological order to finally represent dynamic pulse waves by a normalized PPG signal.

With reference to the PPG signal analysis principle, we also find that, in the continuous heartbeat process, the skin surface of a test subject absorbs the energy of red light and green light in natural light in the same trend as a specific light source of a conventional PPG acquisition device. So, other media may be used to obtain the normalized PPG signal as long as the media can reflect the change trend of red and green light on the skin surface within a time period.

Based on the above finding, different from conventional PPG signal acquisition devices, the embodiments of the invention have no specific light requirements, do not irradiate the skin surface with a specific light source and do not acquire the light intensity with a specific sensor. In the embodiments of the invention, natural light is adopted (even if a strong light source such as a flashlight is used, the strong light source is merely used to increase the light intensity rather than specifying the light property), a common photographing device (such as a camera of a mobile terminal) is used to photograph a designated skin surface (such as the finger, the earlobe, the neck or the forehead) of the test subject, and frame images are extracted from acquired video data to generate a frame image sequence. In the embodiments of the invention, these continuous frame images are used as a medium to capture the change trend of red and green lights, which is specifically implemented as follows: weighted statistics is performed on red and green pixel values in each frame image to generate original red and green signal points of the frame image at a corresponding time, and corresponding filtering and denoising processing is performed on the original red and green signals to generate red and green data channel values of the PPG signal.

In the embodiments of the invention, a local photographing device is provided to record a video to acquire a PPG signal, and a local display device is used to realize synchronous display; meanwhile, a remote server is provided to deduce a PPG signal from uploaded video data, and then analyzed PPG signal data is saved in a medical database, so that data backup and shearing are fulfilled.

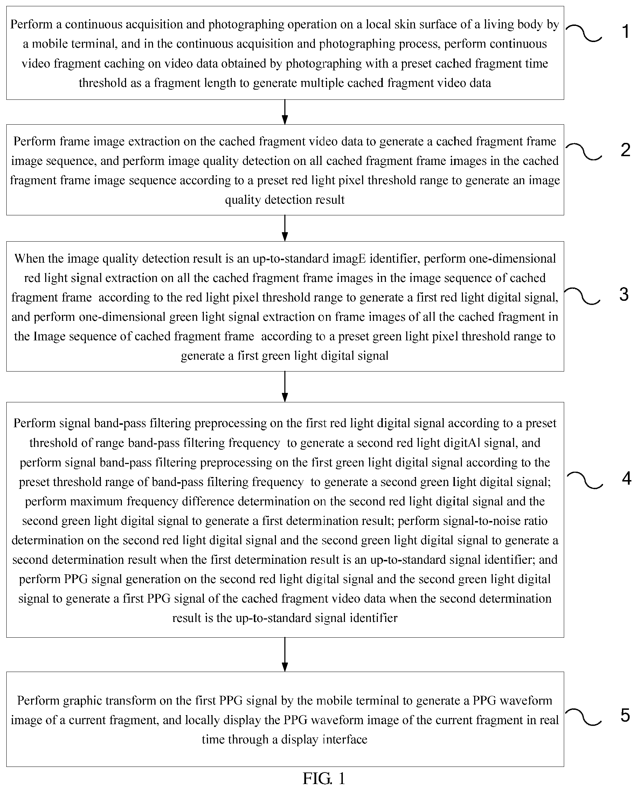

As shown in FIG. 1 which is a schematic diagram of a method for generating PPG signals according to Embodiment 1 of the invention, the method comprises the following steps:
Step 1, a continuous acquisition and photographing operation is performed on a local skin surface of a living body by a mobile terminal, and in the continuous acquisition and photographing process, continuous video fragment caching is performed on video data obtained by photographing with a preset cached fragment time threshold as a fragment length to generate multiple cached fragment video data.

Here, any mobile terminals provided with a camera and having a video recording function in daily life may be used as the video recording device in this embodiment of the invention; and before photographing, a flashlight of the mobile terminal is turned on and is kept on normally to meet the requirement for a stable illuminating light source;
During photographing, the local skin surface of the photographed living body should be slightly pressed against the camera of the mobile terminal; if the flashlight and the camera of the mobile terminal are close to each other, the skin surface covers both the flashlight and the camera; or, if the flashlight and the camera of the mobile terminal are away from each other, the skin surface only covers the camera; and in the photographing process, the skin surface should be kept static, and force should be applied uniformly to the skin surface.

Step 2, frame image extraction is performed on the cached fragment video data to generate an image sequence of cached fragment frame, and image quality detection is performed on all cached fragment frame images in the image sequence of cached fragment frame according to a preset red light pixel threshold range to generate an image quality detection result;
Wherein, the image sequence of cached fragment frame comprises multiple cached fragment frame images;
Step 2 comprises: Step 21, data fragment extraction is sequentially performed on the cached fragment video data according to a preset cached fragment frame data length to obtain multiple data fragments, the multiple cached fragment frame images are generated according to the multiple data fragments, and all the cached fragment frame images are sorted in order to generate the image sequence of cached fragment frame ; and
Step 22, cached fragment frame image extraction is sequentially performed on the image sequence of cached fragment frame in chronological order to generate a current cached fragment frame image; a total number of pixels in the current cached fragment frame image is calculated to generate a total pixel number, and a total number of pixels with a pixel value within the red light pixel threshold range is calculated to generate a total red light pixel number; a red proportion parameter of a current frame is generated according to a ratio of the total red light pixel number to the total pixel number; image quality detection is performed on the red proportion parameter of the current frame according to a preset red proportion lower threshold; when the red proportion parameter of the current frame is less than the red proportion lower threshold, the image quality detection result is set as a not-up-to-standard image identifier, and extraction of cached fragment frame image performed on the image sequence of cached fragment frame is stopped; or, when the red proportion parameter of the current frame is greater than or equal to the red proportion lower threshold, the image quality detection result is set as an up-to-standard image identifier, and extraction of cached fragment frame image continues to be performed on the image sequence of cached fragment frame.

Here, in Step 21-Step 22, sub-frame images in video data are scanned sequentially to check whether the proportion of red pixels in all the sub-frame images is up to standard. If the skin surface completely covers the camera in the photographing process, most pixels in the sub-frame images in a video are red pixels; on the contrary, if the skin surface is not pressed against the camera or is moved away from the camera, the number of red pixels will be decreased. When the currently scanned sub-frame image is not to up standard, the current full scanning process is ended, and the image quality detection result is set as a not-up-to-standard image identifier. If the image quality detection result is the not-up-to-standard image identifier, an upper application will perform the following operation: stopping the continuous acquisition and photographing operation and generating out-of-skin surface photographing error information, transferring the out-of-skin surface photographing error information to the mobile terminal, and displaying, by the mobile terminal, the out-of-skin surface photographing error information as warning information through a display interface.
Step 3, when the image quality detection result is an up-to-standard image identifier, one-dimensional red light signal extraction is performed on all the cached fragment frame images in the image sequence of cached fragment frame according to the red light pixel threshold range to generate a first red light digital signal, and one-dimensional green light signal extraction is performed on all the cached fragment frame images in the image sequence of cached fragment frame according to a preset green light pixel threshold range to generate a first green light digital signal;
Step 3 comprises: Step 31, when the image quality detection result is the up-to-standard image identifier, the first red light digital signal is initialized to be null, the first green light digital signal is initialized to be null, a first index is initialized to 1, and a first total number is initialized to a total number of the cached fragment frame images in the image sequence of cached fragment frame;
Step 32, a first-index frame image is set as the cached fragment frame image, corresponding to the first index, in the image sequence of cached fragment frame ;
Step 33, all pixels, meeting the red light pixel threshold range, in the first-index frame image are collected to generate a red pixel set, a total number of pixels included in the red pixel set is calculated to generate a red pixel total number, the sum of pixel values of all the pixels in the red pixel set is calculated to generate a red pixel value sum, and first-index frame red light channel data is generated according to a quotient obtained by dividing the red pixel value sum by the red pixel total number; and the first-index frame red light channel data is added into the first red light digital signal as signal point data;
For example, the total number of all pixels, meeting the red light pixel threshold range, in a first frame of image are extracted to generate a red pixel total number (because the degrees of reflection and transmission of different parts under the irradiation of a light source are different due to their internal structure or blood vessels, the light transmittances will be different, the shades of red pixels in the recorded video will also be different, so the pixel threshold range is adopted), and the sum of pixel values of all the pixels, meeting the red light pixel threshold range, in the first frame of image is calculated to generate a red pixel value sum, and the first-index frame red channel data = the red pixel value sum / the total number of red pixels;
Step 34, all pixels, meeting the green light pixel threshold range, in the first-index frame image are collected to generate a green pixel set, a total number of pixels included in the green pixel set is calculated to generate a total number of green pixel, the sum of pixel values of all the pixels in the green pixel set is calculated to generate a green pixel value sum, and first-index frame green light channel data is generated according to a quotient obtained by dividing the green pixel value sum by the green pixel total number; and the first-index frame green light channel data is added into the first green light digital signal as signal point data;
For example, the total number of all pixels points, meeting the green light pixel threshold range, in a first frame of image are extracted to generate a green pixel total number (because the degrees of reflection and transmission of different parts under the irradiation of a light source are different due to their internal structure or blood vessels, the light transmittances will be different, the shades of green pixels in the recorded video will also be different, so the pixel threshold range is adopted), and the sum of pixel values of all the pixels, meeting the green light pixel threshold range, in the first frame of image is calculated to generate a green pixel value sum, and the first-index frame green channel data = the sum of green pixel values / the total number of green pixels;
Step 35, the first index is increased by 1;
Step 36, whether the first index is greater than the first total number is determined; if the first index is less than or equal to the first total number, Step 32 is performed; or, if the first index is greater than the first total number, Step 37 is performed; and
Step 37, the first red light digital signal is transferred to an upper processing process as a one-dimensional red light signal extraction result, and the first green light digital signal is transferred to the upper processing process as a one-dimensional green light signal extraction result.

Here, in Step 3, information of two lights, red light and green light, in all cached fragment frame images in the image sequence of cached fragment frame converted from fragment video data is extracted; light signals are extracted in the following way: weighted average calculation is performed on specific pixels in one frame image to obtain a pixel average that is used to represent the color channel data of the corresponding light in the frame image; and each frame image in the video is processed in the same way in chronological order to obtain two segments of one-dimensional digital signals: first red light digital signal and first green light digital signal.

Step 4, signal band-pass filtering preprocessing is performed on the first red light digital signal according to a preset band-pass filtering frequency threshold range to generate a second red light digital signal, and signal band-pass filtering preprocessing is performed on the first green light digital signal according to the preset band-pass filtering frequency threshold range to generate a second green light digital signal; maximum frequency difference determination is performed on the second red light digital signal and the second green light digital signal to generate a first determination result; signal-to-noise ratio determination is performed on the second red light digital signal and the second green light digital signal to generate a second determination result when the first determination result is an up-to-standard signal identifier; and PPG signal generation is performed on the second red light digital signal and the second green light digital signal to generate a first PPG signal of the cached fragment video data when the second determination result is the up-to-standard signal identifier.

Wherein, signal band-pass filtering preprocessing is performed on the first red light digital signal according to the preset band-pass filtering frequency threshold range to generate the second red light digital signal, and signal band-pass filtering preprocessing is performed on the first green light digital signal according to the preset band-pass filtering frequency threshold range to generate the second green light digital signal;
It includes Step 41, according to the band-pass filtering frequency threshold range, digital signal filtering processing is performed on low-frequency noise signal points with a signal frequency lower than the band-pass filtering frequency threshold range and high-frequency noise signal points with a signal frequency higher than the band-pass filtering frequency threshold range in the first red light digital signal to generate the second red light digital signal;
Step 42, according to the band-pass filtering frequency threshold range, digital signal filtering processing is performed on low-frequency noise signal points with a signal frequency lower than the band-pass filtering frequency threshold range and high-frequency noise signal points with a signal frequency higher than the band-pass filtering frequency threshold range in the first green light digital signal to generate the second green light digital signal.

Here, in Step 41and Step 42, signal preprocessing, namely denoising, is performed on the two light digital signals extracted from the video data. In Embodiment 1, band-pass filtering is used for denoising, that is, a band-pass filtering frequency threshold range is preset, and signals, interference and noise lower or higher than the band-pass filtering frequency threshold range are restrained based on the band-pass filtering principle. Generally, the band-pass filtering frequency threshold range is 0.5-10 THz. When some mobile terminals are used for band-pass filtering, a finite impulse response (FIR) filtering module is used;
Wherein, maximum frequency difference determination is performed on the second red light digital signal and the second green light digital signal to generate the first determination result, signal-to-noise ratio determination is performed on the second red light digital signal and the second green light digital signal to generate the second determination result when the first determination result is the up-to-standard signal identifier, and PPG signal generation is performed on the second red light digital signal and the second green light digital signal to generate the first PPG signal of the cached fragment video data when the second determination result is the up-to-standard signal identifier;
It includes Step 43, digital signal time domain-frequency domain conversion is performed on the second red light digital signal through discrete Fourier transform to generate a red light frequency domain signal, digital signal time domain-frequency domain conversion is performed on the second green light digital signal through discrete Fourier transform to generate a green light frequency domain signal; a maximum-energy frequency is extracted from the red light frequency domain signal to generate a maximum red light frequency, and a maximum-energy frequency is extracted from the green light frequency domain signal to generate a maximum green light frequency; a frequency difference between the maximum red light frequency and the maximum green light frequency is calculated to generate a maximum red-green frequency difference; when the maximum red-green frequency difference does not exceed a preset maximum frequency difference threshold range, the first determination result is set as the up-to-standard signal identifier; or, when the maximum red-green frequency difference exceeds the maximum frequency difference threshold range, the first determination result is set as the not-up-to-standard signal identifier;
Here, frequency domain signals of the second red light digital signal and the second green light digital signal are obtained through discrete Fourier transform first; maximum-energy frequencies are obtained according to the frequency domain signals (generally, this frequency corresponds to the heart rate); whether the maximum-energy frequencies of the two digital signals are consistent is checked; if an error is within an allowable error range, the first determination result is set as the up-to-standard signal identifier; if the error is large, the first determination result is set as the not-up-to-standard signal identifier, and an upper application will perform the following operation: stopping the continuous acquisition and photographing operation and the PPG signal generation process and generating signal quality error information, transferring the signal quality error information to the mobile terminal, and displaying, by the mobile terminal, the signal quality error information as warning information through a display interface.

Step 44, when the first determination result is the up-to-standard signal identifier, according to a preset band-stop filtering frequency threshold range, valid signal points with a signal frequency meeting the band-stop filtering frequency threshold range are removed from the second red light digital signal through multi-order Butterworth band-stop filtering to generate a red light noise signal, and valid signal points with a signal frequency meeting the band-stop filtering frequency threshold range are removed from the second green light digital signal through multi-order Butterworth band-stop filtering to generate a green light noise signal;
Step 45, signal energy of the second red light digital signal is calculated to generate red light signal energy, signal energy of the red light noise signal is calculated to generate red light noise energy, valid red light signal energy is generated according to a difference between the red light signal energy and the red light noise energy, and a red light signal-to-noise ratio is generated according to a ratio of the valid red light signal energy to the red light noise energy;
Step 46, signal energy of the second green light digital signal is calculated to generate green light signal energy, signal energy of the green light noise signal is calculated to generate green light noise energy, valid green light signal energy is generated according to a difference between the green light signal energy and the green light noise energy, and a green light signal-to-noise ratio is generated according to a ratio of the valid green light signal energy to the green light noise energy;
Step 47, when the red light signal-to-noise ratio and the green light signal-to-noise ratio are both less than a preset signal-to-noise threshold, the second determination result is set as the not-up-to-standard signal identifier; or, when any one of the red light signal-to-noise ratio and the green light signal-to-noise ratio is greater than or equal to the signal-to-noise threshold, the second determination result is set as the up-to-standard signal identifier;
Here, in Step 44-Step 47, secondary filtering is performed on red and green lights: the secondary filtering is band-stop filtering, that is, signals within the band-stop filtering frequency threshold range are restrained, and specifically, multi-order Butterworth band-stop filtering (such as, four-order Butterworth band-stop filtering, or one-order Butterworth band-stop filtering) is adopted; through band-stop filtering, noise and interference signals are reserved to generate noise signals, and then valid signals and the noise signals are calculated to generate signal-to-noise ratios; whether the red and green light digital signals are up to standard are recognized finally according to the signal-to-noise ratios; a reference parameter for determining whether the red and green light digital signals are up to standard is the second determination result; and if the second determination result is a not-up-to-standard signal identifier, an upper application will perform the following operation: stopping the continuous acquisition and photographing operation and the PPG signal generation process and generating signal quality error information, transferring the signal quality error information to the mobile terminal, and displaying, by the mobile terminal, the signal quality error information as warning information through a display interface.

Step 48, when the second determination result is the up-to-standard signal identifier, a red light digital signal of the first PPG signal is set as the second red light digital signal, and a green light digital signal of the first PPG signal is set as the second green light digital signal;
Wherein, the first PPG signal comprises the red light digital signal and the green light digital signal.

Step 5, graphic transform is performed on the first PPG signal by the mobile terminal to generate a PPG waveform image of a current fragment, and the PPG waveform image of the current fragment is locally displayed in real time through a display interface.

Here, cached fragments will not be too long in actual application, so the cached fragments can be analyzed rapidly, the first PPG signal generated by analyzing the cached fragments can be displayed rapidly, and users can ignore the time delay in this process, that is, users can synchronously observe the heart pressure change reflected by the PPG waveform and the PPG state while photographing themselves by a mobile terminal, and medical data analysis software installed on the mobile terminal can synchronously start relevant data analysis and calculation locally by means of the data.

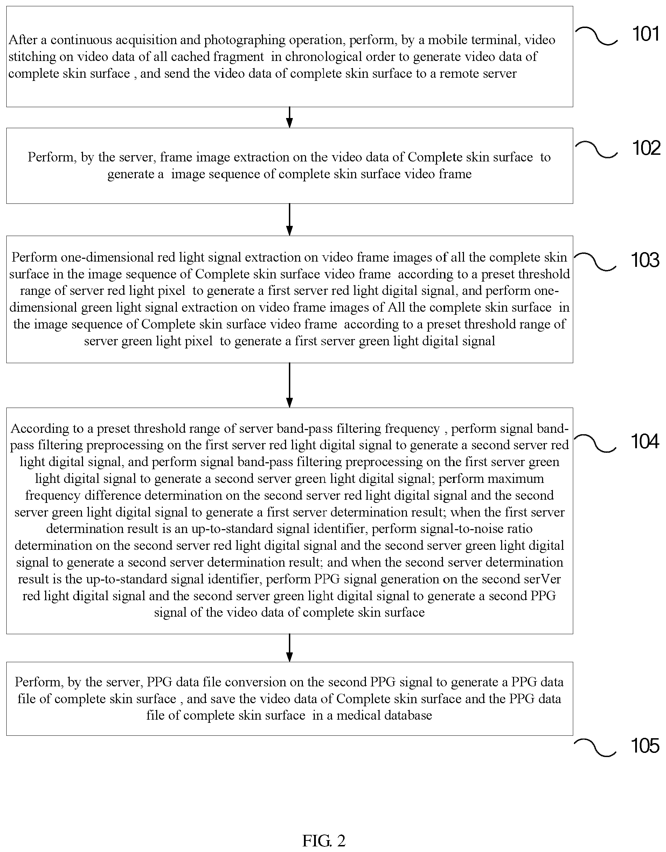

As shown in FIG. 2 which is a schematic diagram of a method for generating PPG signals according to Embodiment 2 of the invention, the method comprises the following steps:
Step 101, after a continuous acquisition and photographing operation, a mobile terminal performs video stitching on all cached fragment video data in chronological order to generate video data of complete skin surfaceand sends the video data of complete skin surfaceto a remote server.

Here, assume the mobile terminal locally generates 10 pieces of cached fragment video data which are respectively cached fragment video data 1, cached fragment video data 2, ..., and cached fragment video data 10, the video data of complete skin surfaceis cached fragment video data 1 + cached fragment video data 2 + ... + cached fragment video data 10.

Step 102, the server performs frame image extraction on the video data of complete skin surfaceto generate an image sequence of complete skin surface video frame;
Wherein, the image sequence of complete skin surface video frame comprises multiple complete skin surface video frame images.

Assume the time of each piece of cached fragment video data is 1s and the number of sub-frame images within 1s is 24, the video data of complete skin surface comprises 10 pieces of cached fragment video data, each fragment comprises 24 frames, and the complete skin surface video frame image sequence comprises 10*24=240 complete skin surface video frame images.

Here, different from the mobile terminal which compares data while acquiring the data, the server performs PPG signal extraction and reproduction on complete data acquired and uploaded by the mobile terminal, regards the uploaded video data as data with up-to-standard image quality that has been subjected to local colored pixel proportion comparison, and thus will no perform colored pixel proportion comparison.
Step 103, one-dimensional red light signal extraction is performed on all the complete skin surface video frame images in the image sequence of complete skin surface video frame according to a preset server red light pixel threshold range to generate a first server red light digital signal, and one-dimensional green light signal extraction is performed on all the complete skin surface video frame images in the image sequence of complete skin surface video frame according to a preset server green light pixel threshold range to generate a first server green light digital signal;
Step 103 comprises: Step 1031, the first server red light digital signal is initialized to be null, the first server green light digital signal is initialized to be null, a second index is initialized to 1, and a second total number is initialized to a total number of the complete skin surface video frame images in the image sequence of complete skin surface video frame;
Step 1032, the complete skin surface video frame image, corresponding to the second index, is extracted from the image sequence of complete skin surface video frame to generate a second-index frame image;
Step 1033, all pixels, meeting a server red light pixel threshold range, in the second-index frame image are collected, and weighted average calculation is performed on pixel values of the collected pixels to generate a second-index frame red light pixel weighted average;
Here, the weighted average calculation adopts a common averaging method. For example, the total number of all pixels, meeting the server red light pixel threshold range, in a first frame of image are extracted to generate a red pixel total number (because the degrees of reflection and transmission of different parts under the irradiation of a light source are different due to their internal structure or blood vessels, the light transmittances will be different, the shades of red pixels in the recorded video will also be different, so the pixel threshold range is adopted), and the sum of pixel values of all the pixels, meeting the red light pixel threshold range, extracted from the first frame of image is calculated to generate a red pixel value sum, and the second-index frame red light pixel weighted average = the red pixel value sum / the red pixel total number;
Here, because this step is performed by the server which has a calculation capacity greater than that of the mobile terminal, a server red light pixel threshold range and a server green light pixel threshold range adopted by the server may be higher in precision than a red light pixel threshold range and a green light pixel threshold range adopted by the mobile terminal or be the same as the red light pixel threshold range and the green light pixel threshold range adopted by the mobile terminal, which will be specifically set by users in actual application;
Step 1034, all pixels, meeting a server green light pixel threshold range, in the second-index frame image are collected, and weighted average calculation is performed on pixel values of the collected pixels to generate a second-index frame green light pixel weighted average;
Here, the weighted average calculation adopts a common averaging method. For example, the total number of all pixels, meeting the server green light pixel threshold range, in a first frame of image are extracted to generate a total number of green pixel (because the degrees of reflection and transmission of different parts under the irradiation of a light source are different due to their internal structure or blood vessels, the light transmittances will be different, the shades of green pixels in the recorded video will also be different, so the pixel threshold range is adopted), and the sum of pixel values of all the pixels, meeting the green light pixel threshold range of the server, extracted from the first frame of image is calculated to generate a green pixel value sum, and the weighted average of second-index frame green light pixel = the green pixel value sum / the total number of green pixel;
Step 1035, signal points are added into the first server red light pixel digital signal according to the second-index frame red light pixel weighted average, and signal points are added into the first server green light pixel digital signal according to the second-index frame green light pixel weighted average;
Step 1036, the second index is increased by 1;
Step 1037, whether the second index is greater than the second total number is determined; if the second index is less than or equal to the second total number, Step 1032 is performed; or, if the second index is greater than the second total number, Step 104 is performed.

Here, in Step 1031-Step 1037, information of two lights, red light and green light, in all complete skin surface video frame images in the image sequence of complete skin surface video frame converted from the video data of complete skin surface is extracted; light signals are extracted in the following way: weighted average calculation is performed on specific pixels in one frame image to obtain a pixel average that is used to represent the color channel data of the corresponding light in the frame image; and each frame image in the video is processed in the same way in chronological order to obtain two segments of one-dimensional digital signals: first server red light digital signal and first server green light digital signal.

Step 104, according to a preset server band-pass filtering frequency threshold range, signal band-pass filtering preprocessing is performed on the first server red light digital signal to generate a second server red light digital signal, and signal band-pass filtering preprocessing is performed on the first server green light digital signal to generate a second server green light digital signal; maximum frequency difference determination is performed on the second server red light digital signal and the second server green light digital signal to generate a first server determination result; when the first server determination result is an up-to-standard signal identifier, signal-to-noise ratio determination is performed on the second server red light digital signal and the second server green light digital signal to generate a second server determination result; and when the second server determination result is the up-to-standard signal identifier, PPG signal generation is performed on the second server red light digital signal and the second server green light digital signal to generate a second PPG signal of the complete skin surface video data;
Wherein, according to the preset server band-pass filtering frequency threshold range, signal band-pass filtering preprocessing is performed on the first server red light digital signal to generate the second server red light digital signal, and signal band-pass filtering preprocessing is performed on the first server green light digital signal to generate the second server green light digital signal;
It includes, Step 1041, according to the server band-pass filtering frequency threshold range, digital signal filtering processing is performed on low-frequency noise signal points with a signal frequency lower than the band-pass filtering frequency threshold range and high-frequency noise signal points with a signal frequency higher than the server band-pass filtering frequency threshold range in the first server red light digital signal to generate the second server red light digital signal;
Step 1042, according to the server band-pass filtering frequency threshold range, digital signal filtering processing is performed on low-frequency noise signal points with a signal frequency lower than the band-pass filtering frequency threshold range and high-frequency noise signal points with a signal frequency higher than the server band-pass filtering frequency threshold range in the first server green light digital signal to generate the second server green light digital signal;
Here, in Step 1041-Step 1042, signal preprocessing, namely denoising, is performed on the two light digital signals extracted from the skin surface video data. In Embodiment 2, band-pass filtering is used for denoising, that is, a band-pass filtering frequency threshold range is preset, and signals, interference and noise lower or higher than the band-pass filtering frequency threshold range are restrained based on the band-pass filtering principle;
Here, because this step is performed by the server which has a calculation capacity greater than that of the mobile terminal, the server band-pass filtering frequency threshold range adopted by the server may be wider than a band-pass filtering frequency threshold range adopted by the mobile terminal or be the same as the band-pass filtering frequency threshold range adopted by the mobile terminal, which will be specifically set by users in actual application;
Wherein, maximum frequency difference determination is performed on the second server red light digital signal and the second server green light digital signal to generate the first server determination result, signal-to-noise ratio determination is performed on the second server red light digital signal and the second server green light digital signal to generate the second server determination result when the first server determination result is the up-to-standard signal identifier, and PPG signal generation is performed on the second server red light digital signal and the second server green light digital signal to generate the second PPG signal of the cached fragment video data when the second server determination result is the up-to-standard signal identifier;
Step 1043, digital signal time domain-frequency domain conversion is performed on the second server red light digital signal through discrete Fourier transform to generate a server red light frequency domain signal, digital signal time domain-frequency domain conversion is performed on the second server green light digital signal through discrete Fourier transform to generate a server green light frequency domain signal; a maximum-energy frequency is extracted from the server red light frequency domain signal to generate a maximum server red light frequency, and a maximum-energy frequency is extracted from the server green light frequency domain signal to generate a maximum server green light frequency; a frequency difference between the maximum server red light frequency and the maximum server green light frequency is calculated to generate a maximum server red-green frequency difference; when the maximum server red-green frequency difference does not exceed a preset maximum server frequency difference threshold range, the first server determination result is set as the up-to-standard signal identifier; or, when the maximum server red-green frequency difference exceeds the maximum server frequency difference threshold range, the first server determination result is set as the not-up-to-standard signal identifier;
Here, frequency domain signals of the second server red light digital signal and the second server green light digital signal are obtained through discrete Fourier transform first; maximum-energy frequencies are obtained according to the frequency domain signals (generally, this frequency corresponds to the heart rate); whether the maximum-energy frequencies of the two digital signals are consistent is checked; if an error is within an allowable error range, analysis is performed continuously; or, if the error is large, the signal quality is regarded as poor, and the server reminds users or an upper application of the poor signal quality through a display interface or a customized error parameter;
Step 1044, when the first server determination result is the up-to-standard signal identifier, according to a preset server band-stop filtering frequency threshold range, valid signal points with a signal frequency meeting the server band-stop filtering frequency threshold range are removed from the second server red light digital signal through multi-order Butterworth band-stop filtering to generate a server red light noise signal, and valid signal points with a signal frequency meeting the server band-stop filtering frequency threshold range are removed from the server second green light digital signal through multi-order Butterworth band-stop filtering to generate a server green light noise signal;
Step 1045, signal energy of the second server red light digital signal is calculated to generate server red light signal energy, signal energy of the server red light noise signal is calculated to generate server red light noise energy, server valid red light signal energy is generated according to a difference between the server red light signal energy and the server red light noise energy, and a server red light signal-to-noise ratio is generated according to a ratio of the server valid red light signal energy to the server red light noise energy;
Step 1046, signal energy of the second server green light digital signal is calculated to generate server green light signal energy, signal energy of the server green light noise signal is calculated to generate server green light noise energy, valid server green light signal energy is generated according to a difference between the server green light signal energy and the server green light noise energy, and a server green light signal-to-noise ratio is generated according to a ratio of the server valid green light signal energy to the server green light noise energy;
Step 1047, if the server red light signal-to-noise ratio and the server green light signal-to-noise ratio are both less than a preset server signal-to-noise threshold, the second server determination result is set as the not-up-to-standard signal identifier; or, if any one of the server red light signal-to-noise ratio and the server green light signal-to-noise ratio is greater than or equal to the server signal-to-noise threshold, the second server determination result is set as the up-to-standard signal identifier;
Here, in Step 1043-Step 1047, secondary filtering is performed on red and green lights: the secondary filtering is band-stop filtering, that is, signals within the band-stop filtering frequency threshold range are restrained, and specifically, multi-order Butterworth band-stop filtering (such as, four-order Butterworth band-stop filtering or one-order Butterworth band-stop filtering) is adopted; through band-stop filtering, noise and interference signals are reserved to generate noise signals, and then valid signals and the noise signals are calculated to generate signal-to-noise ratios; whether the red and green light digital signals are up to standard are recognized finally according to the signal-to-noise ratios; and if the signal quality is not up to standard, the server reminds users or an upper application that the signal quality is not up to standard through a display interface or a customized error parameter;
Here, because this step is performed by the server which has a calculation capacity greater than that of the mobile terminal, the order of multi-order Butterworth band-stop filtering adopted by the server may be greater than the order of multi-order Butterworth band-stop filtering adopted by the mobile terminal (for example, the mobile terminal adopts one-order Butterworth band-stop filtering, and the server adopts four-order or higher-order Butterworth band-stop filtering), or be the same as the order of multi-order Butterworth band-stop filtering adopted by the mobile terminal, which will be specifically set by users in actual application;
Step 1048, when the second server determination result is the up-to-standard signal identifier, a red light digital signal of the second PPG signal is set as the second server red light digital signal, and a green light digital signal of the second PPG signal is set as the second server green light digital signal;
Wherein, the second PPG signal comprises the red light digital signal and the green light digital signal.

Step 105, the server performs PPG data file conversion on the second PPG signal to generate a PPG data file of complete skin surface , and the video data of complete skin surface and the PPG data file of complete skin surface are saved in a medical database.

Here, through Step 101 to Step 105, remote backup of original video data acquired by a local mobile device is realized by the server, the video data is further analyzed by the server to generate a corresponding PPG signal which is saved in the medical database, and all software, hardware, systems and networks connected to the database can use the data repeatedly for various applications.

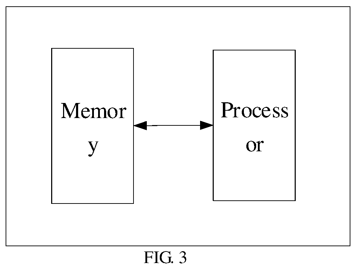

As shown in FIG. 3 which is a structural diagram of a device for generating PPG signals provided by Embodiment 3 of the invention. The equipment comprises a processor and a memory. The memory may be connected to the processor through a bus. The memory may be a nonvolatile memory such as a hard disk drive and a flash memory, and a software program and an equipment drive program are stored in the memory. The software program can implement all functions of the method provided by the embodiments of the invention, and the equipment drive program may be a network and interface drive program. The processor is used to execute the software program, and when the software program is executed, the method provided by the embodiments of the invention is implemented.

It should be noted that the embodiments of the invention further provide a computer-readable storage medium having a computer program stored therein, and when the computer program is executed by a processor, the method provided by the embodiments of the invention is implemented.

The embodiments of the invention further provide a computer program product comprising instructions. When the computer program product runs on a computer, a processor implements the method mentioned above.

The embodiments of the invention provide a method and device for generating PPG signals. By adoption of the method provided by the embodiments of the invention, a PPG signal front-end acquisition device is simplified; a video is recoded and is analyzed to generate a PPG signal, so that the data generation process no longer depends on specific sensor analysis modules; and remote backup of the recoded video and data analysis and processing of the remote video are provided, so that the data sharing degree of an upper application is improved, and the upper application can establish a large PPG database.

Those skilled should further appreciate that the units and arithmetic steps described in conjunction with the embodiments in this specification may be implemented by electronic hardware, computer software, or a combination of these two. To clearly explain the interchangeability of hardware and software, the components and steps of illustrative embodiments have been generally described according to their functions. Whether these functions are implemented by hardware or software depends on specific applications and design constraints of the technical solutions. For each specific application, those skilled may implement these functions in different ways, which should not be construed as exceeding the scope of the invention.

The steps of the method or algorithm described in the embodiments in this specification may be implemented by hardware, software modules executed by a processor, or a combination of these two. The software modules may be configured in a random access memory (RAM), a memory, a read-only memory (ROM), an electrically programmable ROM, an electrically erasable and programmable ROM, a register, a hard disk, a removable disk, a CD-ROM, or a storage medium in any other forms in the art.

The purposes, technical solutions and beneficial effects of the invention are described in further detail with reference to the above specific implementations. It should be understood that the above implementations are merely specific ones of the invention, and are not used to limit the protection scope of the invention, which is defined by the claims.

## Claims

1. A method for generating PPG signals, comprising:
performing a continuous acquisition and photographing operation on a local skin surface of a living body by a mobile terminal, and during the continuous acquisition and photographing process, performing continuous video fragment caching on video data obtained by photographing with a preset cached fragment time threshold as a fragment length to generate multiple cached fragment video data (1);
performing frame image extraction on the cached fragment video data to generate a image sequence of cached fragment frame, and performing image quality detection on all cached fragment frame images in the image sequence of cached fragment frame according to a preset red light pixel threshold range to generate an image quality detection result (2), wherein the image sequence of cached fragment frame comprises multiple cached fragment frame images;
when the image quality detection result is an up-to-standard image identifier, performing one-dimensional red light signal extraction on all the cached fragment frame images in the image sequence of cached fragment frame according to the red light pixel threshold range to generate a first red light digital signal, and performing one-dimensional green light signal extraction on all the cached fragment frame images in the image sequence of cached fragment frame according to a preset green light pixel threshold range to generate a first green light digital signal (3); and
performing signal band-pass filtering preprocessing on the first red light digital signal according to a preset band-pass filtering frequency threshold range to generate a second red light digital signal, and performing signal band-pass filtering preprocessing on the first green light digital signal according to the preset band-pass filtering frequency threshold range to generate a second green light digital signal; performing maximum frequency difference determination on the second red light digital signal and the second green light digital signal to generate a first determination result; performing signal-to-noise ratio determination on the second red light digital signal and the second green light digital signal to generate a second determination result when the first determination result is an up-to-standard signal identifier; and performing PPG signal generation on the second red light digital signal and the second green light digital signal to generate a first PPG signal of the cached fragment video data when the second determination result is the up-to-standard signal identifier (4);
**characterized in that**
the step of when the image quality detection result is an up-to-standard image identifier, performing one-dimensional red light signal extraction on all the cached fragment frame images in the image sequence of cached fragment frame according to the red light pixel threshold range to generate a first red light digital signal, and performing one-dimensional green light signal extraction on all the cached fragment frame images in the image sequence of cached fragment frame according to a preset green light pixel threshold range to generate a first green light digital signal specifically comprises:
Step 51, when the image quality detection result is the up-to-standard image identifier, initializing the first red light digital signal to be null, initializing the first green light digital signal to be null, initializing a first index to 1, and initializing a first total number to a total number of the cached fragment frame images in the image sequence of cached fragment frame;
Step 52, setting a first-index frame image as the cached fragment frame image, corresponding to the first index, in the image sequence of cached fragment frame;
Step 53, collecting all pixels, meeting the red light pixel threshold range, in the first-index frame image to generate a red pixel set, calculating a total number of pixels included in the red pixel set to generate a total number of red pixel, calculating the sum of pixel values of all the pixels in the red pixel set to generate a value sum of red pixel , and generating first-index frame red light channel data according to a quotient obtained by dividing the value sum of red pixel by the total number of red pixel; and adding the first-index frame red light channel data into the first red light digital signal as signal point data;
Step 54, collecting all pixels, meeting the green light pixel threshold range, in the first-index frame image to generate a green pixel set, calculating a total number of pixels included in the green pixel set to generate a total number of green pixel, calculating the sum of pixel values of all the pixels in the green pixel set to generate a value sum of green pixel, and generating first-index frame green light channel data according to a quotient obtained by dividing the green pixel value sum by the total number of green pixel; and adding the first-index frame green light channel data into the first green light digital signal as signal point data;
Step 55, increasing the first index by 1;
Step 56, determining whether the first index is greater than the first total number; if the first index is less than or equal to the first total number, performing Step 52; or, if the first index is greater than the first total number, performing Step 57; and
Step 57, transferring the first red light digital signal to an upper processing process as a one-dimensional red light signal extraction result, and transferring the first green light digital signal to the upper processing process as a one-dimensional green light signal extraction result;
wherein the step of performing signal band-pass filtering preprocessing on the first red light digital signal according to a preset band-pass filtering frequency threshold range to generate a second red light digital signal, and performing signal band-pass filtering preprocessing on the first green light digital signal according to the preset band-pass filtering frequency threshold range to generate a second green light digital signal comprises:
according to the band-pass filtering frequency threshold range, performing digital signal filtering processing on low-frequency noise signal points with a signal frequency lower than the band-pass filtering frequency threshold range and high-frequency noise signal points with a signal frequency higher than the band-pass filtering frequency threshold range in the first red light digital signal to generate the second red light digital signal; and
according to the band-pass filtering frequency threshold range, performing digital signal filtering processing on low-frequency noise signal points with a signal frequency lower than the band-pass filtering frequency threshold range and high-frequency noise signal points with a signal frequency higher than the band-pass filtering frequency threshold range in the first green light digital signal to generate the second green light digital signal.

2. The method for generating PPG signals according to Claim 1, wherein the method further comprises:
performing a real time display on the first PPG signal, and locally displaying PPG waveform image n real time through a display interface (5).

3. The method for generating PPG signals according to Claim 1, wherein the method further comprises:
after the continuous acquisition and photographing operation, performing video stitching on all the cached fragment video data in chronological order by the mobile terminal to generate complete skin surface video data, and sending the video data of complete skin surface to a remote server (101);
performing frame image extraction on the video data of complete skin surface by the server to generate an image sequence of complete skin surface video frame (102), wherein the image sequence of complete skin surface video frame comprises multiple complete skin surface video frame images;
performing one-dimensional red light signal extraction on all the complete skin surface video frame images in the image sequence of complete skin surface video frame according to a preset server red light pixel threshold range to generate a first server red light digital signal, and performing one-dimensional green light signal extraction on all the complete skin surface video frame images in the image sequence of complete skin surface video frame according to a preset server green light pixel threshold range to generate a first server green light digital signal (103);
according to a preset server band-pass filtering frequency threshold range, performing signal band-pass filtering preprocessing on the first server red light digital signal to generate a second server red light digital signal, and performing signal band-pass filtering preprocessing on the first server green light digital signal to generate a second server green light digital signal;
performing maximum frequency difference determination on the second server red light digital signal and the second server green light digital signal to generate a first server determination result; when the first server determination result is the up-to-standard signal identifier,
performing signal-to-noise ratio determination on the second server red light digital signal and the second server green light digital signal to generate a second server determination result; and when the second server determination result is the up-to-standard signal identifier,
performing PPG signal generation on the second server red light digital signal and the second server green light digital signal to generate a second PPG signal of the complete skin surface video data (104); and
performing PPG data file conversion on the second PPG signal by the server to generate a PPG data file of complete skin surface , and saving the video data of complete skin surface and the PPG data file of complete skin surface in a medical database (105).

4. The method for generating PPG signals according to Claim 1, wherein the step of performing frame image extraction on the cached fragment video data to generate a cached fragment frame image sequence, and performing image quality detection on all cached fragment frame images in the image sequence of cached fragment frame according to a preset red light pixel threshold range to generate an image quality detection result comprises:
sequentially performing data fragment extraction on the cached fragment video data according to a preset cached fragment frame data length to obtain multiple data fragments, generating the multiple cached fragment frame images according to the extracted multiple data fragments, and sorting all the cached fragment frame images in order to generate the image sequence of cached fragment frame; and
sequentially performing cached fragment frame image extraction on the image sequence of cached fragment frame in chronological order to generate a current cached fragment frame image; calculating a total number of pixels in the current cached fragment frame image to generate a total pixel number, and calculating a total number of pixels with a pixel value within the red light pixel threshold range to generate a total number of red light pixel; generating a red proportion parameter of a current frame according to a ratio of the total number of red light pixel to the total number of pixels; performing image quality detection on the red proportion parameter of the current frame according to a preset red proportion lower threshold; when the red proportion parameter of the current frame is less than the red proportion lower threshold, setting the image quality detection result as a not-up-to-standard image identifier, and stopping cached fragment frame image extraction performed on the image sequence of cached fragment frame ; or, when the red proportion parameter of the current frame is greater than or equal to the red proportion lower threshold, setting the image quality detection result as the up-to-standard image identifier, and continuing to perform cached fragment frame image extraction on the image sequence of cached fragment frame.

5. The method for generating PPG signals according to Claim 1, wherein the step of performing maximum frequency difference determination on the second red light digital signal and the second green light digital signal to generate a first determination result, performing signal-to-noise ratio determination on the second red light digital signal and the second green light digital signal to generate a second determination result when the first determination result is an up-to-standard signal identifier, and performing PPG signal generation on the second red light digital signal and the second green light digital signal to generate a first PPG signal of the cached fragment video data when the second determination result is the up-to-standard signal identifier comprises:
Step 71, performing digital signal time domain-frequency domain conversion on the second red light digital signal through discrete Fourier transform to generate a red light frequency domain signal, performing digital signal time domain-frequency domain conversion on the second green light digital signal through discrete Fourier transform to generate a green light frequency domain signal; extracting a maximum-energy frequency from the red light frequency domain signal to generate a maximum red light frequency, and extracting a maximum-energy frequency from the green light frequency domain signal to generate a maximum green light frequency; calculating a frequency difference between the maximum red light frequency and the maximum green light frequency to generate a maximum red-green frequency difference; when the maximum red-green frequency difference does not exceed a preset maximum frequency difference threshold range, setting the first determination result as the up-to-standard signal identifier; or, when the maximum red-green frequency difference exceeds the maximum frequency difference threshold range, setting the first determination result as the not-up-to-standard signal identifier;
Step 72, when the first determination result is the up-to-standard signal identifier, according to a preset band-stop filtering frequency threshold range, removing valid signal points with a signal frequency meeting the band-stop filtering frequency threshold range from the second red light digital signal through multi-order Butterworth band-stop filtering to generate a red light noise signal, and removing valid signal points with a signal frequency meeting the band-stop filtering frequency threshold range from the second green light digital signal through multi-order Butterworth band-stop filtering to generate a green light noise signal;
Step 73, calculating signal energy of the second red light digital signal to generate red light signal energy, calculating signal energy of the red light noise signal to generate red light noise energy, generating valid red light signal energy according to a difference between the red light signal energy and the red light noise energy, and generating a red light signal-to-noise ratio according to a ratio of the valid red light signal energy to the red light noise energy;
Step 74, calculating signal energy of the second green light digital signal to generate green light signal energy, calculating signal energy of the green light noise signal to generate green light noise energy, generating valid green light signal energy according to a difference between the green light signal energy and the green light noise energy, and generating a green light signal-to-noise ratio according to a ratio of the valid green light signal energy to the green light noise energy;
Step 75, when the red light signal-to-noise ratio and the green light signal-to-noise ratio are both less than a preset signal-to-noise threshold, setting the second determination result as the not-up-to-standard signal identifier; or, when any one of the red light signal-to-noise ratio and the green light signal-to-noise ratio is greater than or equal to the signal-to-noise threshold, setting the second determination result as the up-to-standard signal identifier;
Step 76, when the second determination result is the up-to-standard signal identifier, setting a red light digital signal of the first PPG signal as the second red light digital signal, and setting a green light digital signal of the first PPG signal as the second green light digital signal, wherein the first PPG signal comprises the red light digital signal and the green light digital signal.

6. The method for generating PPG signals according to Claim 1, wherein the method further comprises:
when the image quality detection result is the not-up-to-standard signal identifier, stopping the continuous acquisition and photographing operation and generating out-of-skin surface error information, then transferring the out-of-skin surface error information to the mobile terminal, and displaying, by the mobile terminal, the out-of-skin surface error information as warning information through the display interface;
when the first determination result is the not-up-to-standard signal identifier, stopping the continuous acquisition and photographing operation and the PPG signal generation process and generating signal quality error information, then transferring the signal quality error information to the mobile terminal, and displaying, by the mobile terminal, the signal quality error information as warning information through the display interface; or
when the second determination result is the not-up-to-standard signal identifier, stopping the continuous acquisition and photographing operation and the PPG signal generation process and generating signal quality error information, then transferring the signal quality error information to the mobile terminal, and displaying, by the mobile terminal, the signal quality error information as warning information through the display interface.

7. An equipment, comprising a memory and a processor, wherein the memory is used to store a program, and the processor is used to implement the method according to any one of Claims 1-6.

8. A computer program product comprising instructions, enabling a computer to implement the method according to any one of Claims 1-6 when running on the computer.

9. A computer-readable storage medium comprising instructions which, when executed by a computer, cause the computer to implement the method according to any one of claims 1-6.

## Patentansprüche

1. Verfahren zum Erzeugen von PPG-Signalen, umfassend:
Durchführen einer kontinuierlichen Erfassungs- und Bildaufnahmeoperation an einer lokalen Hautoberfläche eines lebenden Körpers durch ein mobiles Endgerät und während des kontinuierlichen Erfassungs- und Bildaufnahmeprozesses Durchführen einer kontinuierlichen Videofragment-Zwischenspeicherung von durch die Aufnahme erhaltenen Videodaten mit einem voreingestellten Schwellenwert für die Zwischenspeicherungsdauer als Fragmentlänge, um mehrere zwischengespeicherte Fragment-Videodaten zu erzeugen (1);
Durchführen einer Frame-Bildextraktion an den zwischengespeicherten Fragment-Videodaten, um eine Bildsequenz von zwischengespeicherten Fragment-Frames zu erzeugen, und Durchführen einer Bildqualitätserkennung an allen zwischengespeicherten Fragment-Frame-Bildern in der Bildsequenz von zwischengespeicherten Fragment-Frames gemäß einem voreingestellten Schwellenwertbereich für rote Lichtpixel, um ein Bildqualitätserkennungsergebnis zu erzeugen (2), wobei die Bildsequenz von zwischengespeicherten Fragment-Frames mehrere zwischengespeicherte Fragment-Frame-Bilder umfasst;
wenn das Bildqualitätserkennungsergebnis ein normgerechter Bildidentifikator ist, Durchführen einer eindimensionalen Rotlicht-Signalextraktion an allen zwischengespeicherten Fragment-Frame-Bildern in der Bildsequenz der zwischengespeicherten Fragment-Frame-Bilder gemäß dem Rotlicht-Pixel-Schwellenbereich, um ein erstes Rotlicht-Digitalsignal zu erzeugen, und Durchführung einer eindimensionalen Grünlicht-Signalextraktion an allen zwischengespeicherten Fragment-Frame-Bildern in der Bildsequenz der zwischengespeicherten Fragment-Frame-Bilder gemäß einem voreingestellten Grünlicht-Pixel-Schwellenbereich, um ein erstes Grünlicht-Digitalsignal (3) zu erzeugen; und
Durchführen einer Signalbandpassfilter-Vorverarbeitung an dem ersten Rotlicht-Digitalsignal gemäß einem voreingestellten Bandpassfilter-Frequenzschwellenbereich, um ein zweites Rotlicht-Digitalsignal zu erzeugen, und Durchführen einer Signalbandpassfilter-Vorverarbeitung an dem ersten Grünlicht-Digitalsignal gemäß dem voreingestellten Bandpassfilter-Frequenzschwellenbereich, um ein zweites Grünlicht-Digitalsignal zu erzeugen;
Durchführen einer Bestimmung der maximalen Frequenzdifferenz für das zweite Rotlicht-Digitalsignal und das zweite Grünlicht-Digitalsignal, um ein erstes Bestimmungsergebnis zu erzeugen; Durchführen einer Bestimmung des Signal-Rausch-Verhältnisses für das zweite Rotlicht-Digitalsignal und das zweite Grünlicht-Digitalsignal, um ein zweites Bestimmungsergebnis zu erzeugen, wenn das erste Bestimmungsergebnis ein normgerechtes Signalidentifikationszeichen ist; und Durchführen einer PPG-Signalerzeugung für das zweite Rotlicht-Digitalsignal und das zweite Grünlicht-Digitalsignal, um ein erstes PPG-Signal der zwischengespeicherten Fragment-Videodaten zu erzeugen, wenn das zweite Bestimmungsergebnis das normgerechte Signalidentifikationszeichen ist (4);
**dadurch gekennzeichnet, dass** der Schritt, wenn das Bildqualitätserkennungsergebnis ein normgerechter Bildidentifikator ist, Durchführen einer eindimensionalen Rotlicht-Signalextraktion an allen zwischengespeicherten Fragment-Frame-Bildern in der Bildsequenz der zwischengespeicherten Fragment-Frame-Bilder gemäß dem Rotlicht-Pixel-Schwellenbereich, um ein erstes Rotlicht-Digitalsignal zu erzeugen, und
Durchführung einer eindimensionalen Grünlicht-Signalextraktion an allen zwischengespeicherten Fragment-Frame-Bildern in der Bildsequenz der zwischengespeicherten Fragment-Frame-Bilder gemäß einem voreingestellten Grünlicht-Pixel-Schwellenbereich, um ein erstes Grünlicht-Digitalsignal zu erzeugen, insbesondere umfasst:
Schritt 51: wenn das Bildqualitätserkennungsergebnis der normgerechte Bildidentifikator ist, Initialisieren des ersten Rotlicht-Digitalsignals auf Null, Initialisieren des ersten Grünlicht-Digitalsignals auf Null, Initialisieren eines ersten Index auf 1 und Initialisieren einer ersten Gesamtzahl auf eine Gesamtzahl der zwischengespeicherten Fragment-Frame-Bilder in der Bildsequenz des zwischengespeicherten Fragment-Rahmens;
Schritt 52: Einstellen eines ersten Index-Rahmenbildes als zwischengespeichertes Fragment-Rahmenbild, das dem ersten Index in der Bildsequenz des zwischengespeicherten Fragment-Rahmens entspricht;
Schritt 53: Sammeln aller Pixel, die den Schwellenbereich für rote Lichtpixel erfüllen, im ersten Index-Rahmenbild, um einen Satz roter Pixel zu erzeugen, Berechnen einer Gesamtzahl von Pixeln, die im Satz roter Pixel enthalten sind, um eine Gesamtzahl roter Pixel zu erzeugen, Berechnen der Summe der Pixelwerte aller Pixel im Satz roter Pixel, um eine Wertesumme roter Pixel zu erzeugen, und Erzeugen von Rotlichtkanaldaten des ersten Index-Rahmenbilds gemäß einem Quotienten, der durch Division der Wertesumme der roten Pixel durch die Gesamtzahl der roten Pixel erhalten wird; und Hinzufügen der Rotlichtkanaldaten des ersten Index-Rahmenbilds zum ersten Rotlicht-Digitalsignal als Signalpunktdaten;
Schritt 54: Sammeln aller Pixel, die den Schwellenbereich für grüne Lichtpixel erfüllen, im ersten Index-Rahmenbild, um einen Satz grüner Pixel zu erzeugen, Berechnen einer Gesamtzahl von Pixeln, die im Satz grüner Pixel enthalten sind, um eine Gesamtzahl grüner Pixel zu erzeugen, Berechnen der Summe der Pixelwerte aller Pixel im Satz grüner Pixel, um eine Wertesumme grüner Pixel zu erzeugen, und Erzeugen von Grünlichtkanaldaten des ersten Index-Rahmenbilds gemäß einem Quotienten, der durch Division der Wertesumme der grünen Pixel durch die Gesamtzahl der grünen Pixel erhalten wird; und Hinzufügen der Grünlichtkanaldaten des ersten Index-Rahmenbilds für den Grünlichtkanal zum ersten Grünlicht-Digitalsignal als Signalpunktdaten;
Schritt 55: Erhöhen des ersten Index um 1;
Schritt 56: Bestimmen, ob der erste Index größer als die erste Gesamtzahl ist; wenn der erste Index kleiner oder gleich der ersten Gesamtzahl ist, Ausführen von Schritt 52; oder, wenn der erste Index größer als die erste Gesamtzahl ist, Ausführen von Schritt 57; und
Schritt 57: Übertragen des ersten Rotlicht-Digitalsignals an einen übergeordneten Verarbeitungsprozess als eindimensionales Rotlicht-Signalextraktionsergebnis und Übertragen des ersten Grünlicht-Digitalsignals an den übergeordneten Verarbeitungsprozess als eindimensionales Grünlicht-Signalextraktionsergebnis;
wobei der Schritt des Durchführens einer Signalbandpassfilter-Vorverarbeitung an dem ersten Rotlicht-Digitalsignal gemäß einem voreingestellten Bandpassfilter-Frequenzschwellenbereich, um ein zweites Rotlicht-Digitalsignal zu erzeugen, und
Durchführen einer Signalbandpassfilter-Vorverarbeitung an dem ersten Grünlicht-Digitalsignal gemäß dem voreingestellten Bandpassfilter-Frequenzschwellenbereich, um ein zweites Grünlicht-Digitalsignal zu erzeugen, umfasst:
gemäß dem Bandpassfilter-Frequenzschwellenbereich Durchführen einer digitalen Signalfilterverarbeitung an Niederfrequenz-Rauschsignalpunkten mit einer Signalfrequenz, die niedriger als der Bandpassfilter-Frequenzschwellenbereich ist, und an Hochfrequenz-Rauschsignalpunkten mit einer Signalfrequenz, die höher als der Bandpassfilter-Frequenzschwellenbereich ist, in dem ersten Rotlicht-Digitalsignal, um das zweite Rotlicht-Digitalsignal zu erzeugen; und
gemäß dem Bandpassfilter-Frequenzschwellenbereich Durchführen einer digitalen Signalfilterung an den Niederfrequenz-Rauschsignalpunkten mit einer Signalfrequenz, die niedriger als der Bandpassfilter-Frequenzschwellenbereich ist, und an den Hochfrequenz-Rauschsignalpunkten mit einer Signalfrequenz, die höher als der Bandpassfilter-Frequenzschwellenbereich ist, in dem ersten Grünlicht-Digitalsignal, um das zweite Grünlicht-Digitalsignal zu erzeugen.

2. Verfahren zum Erzeugen von PPG-Signalen gemäß Anspruch 1, wobei das Verfahren ferner umfasst:
Durchführen einer Echtzeitanzeige des ersten PPG-Signals und lokales Anzeigen des PPG-Wellenformbildes in Echtzeit über eine Anzeige-Schnittstelle (5).

3. Verfahren zum Erzeugen von PPG-Signalen gemäß Anspruch **1,** wobei das Verfahren ferner umfasst:
nach der kontinuierlichen Erfassungs- und Bildaufnahmeoperation, Durchführen eines Video-Stitchings aller zwischengespeicherten Fragment-Videodaten in chronologischer Reihenfolge durch das mobile Endgerät, um vollständige Hautoberflächen-Videodaten zu erzeugen, und Senden der Videodaten der vollständigen Hautoberfläche an einen Remote-Server (101);
Durchführen einer Frame-Bildextraktion für die Videodaten der vollständigen Hautoberfläche durch den Server, um eine Bildsequenz vollständiger Hautoberflächen-Videoframes (102) zu erzeugen, wobei die Bildsequenz vollständiger Hautoberflächen-Videoframes mehrere vollständige Hautoberflächen-Videoframe-Bilder umfasst;
Durchführen einer eindimensionalen Rotlicht-Signalextraktion für alle vollständigen Hautoberflächen-Videoframes in der Bildsequenz vollständiger Hautoberflächen-Videoframes gemäß einem voreingestellten Rotlicht-Pixel-Schwellenbereich des Servers, um ein erstes Rotlicht-Digitalsignal des Servers zu erzeugen, und Durchführen einer eindimensionalen Grünlicht-Signalextraktion für alle vollständigen Hautoberflächen-Videoframes in der Bildsequenz vollständiger Hautoberflächen-Videoframes gemäß einem voreingestellten Grünlicht-Pixel-Schwellenbereich des Servers, um ein erstes Grünlicht-Digitalsignal des Servers zu erzeugen (103);
Durchführen gemäß einem voreingestellten Server-Bandpassfilter-Frequenzschwellenbereich eine Signalbandpassfilter-Vorverarbeitung an dem ersten Server-Rotlicht-Digitalsignal, um ein zweites Server-Rotlicht-Digitalsignal zu erzeugen, und Durchführen einer Signalbandpassfilter-Vorverarbeitung an dem ersten Server-Grünlicht-Digitalsignal, um ein zweites Server-Grünlicht-Digitalsignal zu erzeugen;
Durchführen einer Bestimmung der maximalen Frequenzdifferenz für das zweite Server-Rotlicht-Digitalsignal und das zweite Server-Grünlicht-Digitalsignal, um ein erstes Server-Bestimmungsergebnis zu erzeugen; wenn das erste Server-Bestimmungsergebnis das normgerechte Signalidentifikationszeichen ist, Durchführen einer Bestimmung des Signal-Rausch-Verhältnisses für das zweite Server-Rotlicht-Digitalsignal und das zweite Server-Grünlicht-Digitalsignal, um ein zweites Server-Bestimmungsergebnis zu erzeugen; und
wenn das zweite Server-Bestimmungsergebnis das normgerechte Signalidentifikationszeichen ist, Durchführung einer PPG-Signalerzeugung für das Rotlicht-Digitalsignal des zweiten Servers und das Grünlicht-Digitalsignal des zweiten Servers, um ein zweites PPG-Signal der vollständigen Hautoberflächen-Videodaten (104) zu erzeugen; und
Durchführen einer PPG-Daten-Dateikonvertierung für das zweite PPG-Signal durch den Server, um eine PPG-Daten-Datei der gesamten Hautoberfläche zu erzeugen, und Speichern der Videodaten der gesamten Hautoberfläche und der PPG-Daten-Datei der gesamten Hautoberfläche in einer medizinischen Datenbank (105).

4. Verfahren zum Erzeugen von PPG-Signalen gemäß Anspruch 1, wobei der Schritt des Durchführens einer Frame-Bildextraktion an den zwischengespeicherten Fragment-Videodaten, um eine Bildsequenz von zwischengespeicherten Fragment-Frames zu erzeugen, und des Durchführens einer Bildqualitätserkennung an allen zwischengespeicherten Fragment-Frame-Bildern in der Bildsequenz von zwischengespeicherten Fragment-Frames gemäß einem voreingestellten Schwellenwertbereich für rote Lichtpixel, um ein Bildqualitätserkennungsergebnis zu erzeugen, umfasst:
sequentielles Durchführen einer Datenfragmentextraktion aus den zwischengespeicherten Fragment-Videodaten gemäß einer voreingestellten Länge der zwischengespeicherten Fragment-Frame-Daten, um mehrere Datenfragmente zu erhalten, Erzeugen der mehreren zwischengespeicherten Fragment-Frame-Bilder gemäß den extrahierten mehreren Datenfragmenten und Sortieren aller zwischengespeicherten Fragment-Frame-Bilder, um die Bildsequenz der zwischengespeicherten Fragmentbilder zu erzeugen; und
sequentielles Durchführen einer zwischengespeicherten Fragmentbildrahmenextraktion an der Bildsequenz der zwischengespeicherten Fragment-Frame-Bilder in chronologischer Reihenfolge, um ein aktuelles zwischengespeichertes Fragmentbild zu erzeugen;
Berechnen einer Gesamtzahl von Pixeln in dem aktuellen zwischengespeicherten Fragment-Frame, um eine Gesamtpixelzahl zu erzeugen, und Berechnen einer Gesamtzahl von Pixeln mit einem Pixelwert innerhalb des Schwellenwertbereichs für Rotlichtpixel, um eine Gesamtzahl von Rotlichtpixeln zu erzeugen; Erzeugen eines Rotanteilparameters eines aktuellen Frames gemäß einem Verhältnis der Gesamtzahl von Rotlichtpixeln zur Gesamtzahl von Pixeln; Durchführen einer Bildqualitätserkennung für den Rotanteilsparameter des aktuellen Frames gemäß einem voreingestellten unteren Schwellenwert für den Rotanteil; wenn der Rotanteilsparameter des aktuellen Frames kleiner als der untere Schwellenwert für den Rotanteil ist, Festlegen des Bildqualitätserkennungsergebnisses als Kennzeichen für ein nicht-normgerechtes Bild und Beenden der Extraktion zwischengespeicherter Fragment-Frame-Bilder, die für die Bildsequenz zwischengespeicherter Fragment-Frame-Bilder durchgeführt wird; oder, wenn der Rotanteilsparameter des aktuellen Frames größer oder gleich dem unteren Schwellenwert für den Rotanteil ist, Festlegen des Bildqualitätserkennungsergebnisses als Kennzeichen für ein normgerechtes Bild und Fortsetzen der Extraktion von zwischengespeicherten Fragment-Frame-Bilder aus der Bildsequenz des zwischengespeicherten Fragment-Frames.

5. Verfahren zum Erzeugen von PPG-Signalen gemäß Anspruch 1, wobei der Schritt des Durchführens einer Bestimmung der maximalen Frequenzdifferenz zwischen dem zweiten Rotlicht-Digitalsignal und dem zweiten Grünlicht-Digitalsignal, um ein erstes Bestimmungsergebnis zu erzeugen; Durchführen einer Bestimmung des Signal-Rausch-Verhältnisses für das zweite Rotlicht-Digitalsignal und das zweite Grünlicht-Digitalsignal, um ein zweites Bestimmungsergebnis zu erzeugen, wenn das erste Bestimmungsergebnis ein normgerechtes Signalidentifikationszeichen ist; und Durchführen einer PPG-Signalerzeugung für das zweite Rotlicht-Digitalsignal und das zweite Grünlicht-Digitalsignal, um ein erstes PPG-Signal der zwischengespeicherten Fragment-Videodaten zu erzeugen, wenn das zweite Bestimmungsergebnis das normgerechte Signalidentifikationszeichen ist, umfasst:
Schritt 71, Durchführen einer Zeitdomäne-Frequenzdomäne-Umwandlung des zweiten Rotlicht-Digitalsignals durch diskrete Fourier-Transformation, um ein Rotlicht-Frequenzdomänesignal zu erzeugen, Durchführen einer Zeitdomäne-Frequenzdomäne-Umwandlung des zweiten Grünlicht-Digitalsignals durch diskrete Fourier-Transformation,
um ein Grünlicht-Frequenzdomänesignal zu erzeugen; Extrahieren einer Frequenz mit maximaler Energie aus dem Rotlicht- Frequenzdomänesignal, um eine maximale Rotlichtfrequenz zu erzeugen, und Extrahieren einer Frequenz mit maximaler Energie aus dem Grünlicht-Frequenzdomänesignal, um eine maximale Grünlichtfrequenz zu erzeugen;
Berechnen einer Frequenzdifferenz zwischen der maximalen Rotlichtfrequenz und der maximalen Grünlichtfrequenz, um eine maximale Rot-Grün-Frequenzdifferenz zu erzeugen; wenn die maximale Rot-Grün-Frequenzdifferenz einen voreingestellten maximalen Frequenzdifferenzschwellenbereich nicht überschreitet, Setzen des ersten Bestimmungsergebnisses als normgerechtes Signalidentifikationszeichen; oder, wenn die maximale Rot-Grün-Frequenzdifferenz den maximalen Frequenzdifferenzschwellenbereich überschreitet, Setzen des ersten Bestimmungsergebnisses als nicht-normgerechtes Signalidentifikationszeichen;
Schritt 72, wenn das erste Bestimmungsergebnis das normgerechte Signalidentifikationszeichen ist, gemäß einem voreingestellten Bandstopp-Filterfrequenzschwellenbereich, Entfernen gültiger Signalpunkte mit einer Signalfrequenz, die den Bandstopp-Filterfrequenzschwellenbereich erfüllt, aus dem zweiten Rotlicht-Digitalsignal durch mehrstufige Butterworth-Bandstopp-Filterung, um ein Rotlicht-Rauschsignal zu erzeugen, und Entfernen gültiger Signalpunkte mit einer Signalfrequenz, die den Bandstopp-Filterfrequenzschwellenbereich erfüllt, aus dem zweiten Grünlicht-Digitalsignal durch mehrstufige Butterworth-Bandstopp-Filterung, um ein Grünlicht-Rauschsignal zu erzeugen;
Schritt 73: Berechnen der Signalenergie des zweiten Rotlicht-Digitalsignals, um eine Rotlichtsignalenergie zu erzeugen, Berechnen der Signalenergie des Rotlichtrauschsignals, um eine Rotlichtrauschenergie zu erzeugen, Erzeugen einer gültigen Rotlichtsignalenergie entsprechend einer Differenz zwischen der Rotlichtsignalenergie und der Rotlichtrauschenergie und Erzeugen eines Rotlicht-Signal-Rausch-Verhältnisses entsprechend einem Verhältnis der gültigen Rotlichtsignalenergie zur Rotlichtrauschenergie;
Schritt 74: Berechnen der Signalenergie des zweiten Grünlicht-Digitalsignals, um eine Grünlichtsignalenergie zu erzeugen, Berechnen der Signalenergie des Grünlichtrauschsignals, um eine Grünlichtrauschenergie zu erzeugen, Erzeugen einer gültigen Grünlichtsignalenergie entsprechend einer Differenz zwischen der Grünlichtsignalenergie und der Grünlichtrauschenergie und Erzeugen eines Grünlicht-Signal-Rausch-Verhältnisses entsprechend einem Verhältnis der gültigen Grünlichtsignalenergie zur Grünlichtrauschenergie;
Schritt 75: wenn das Rotlicht-Signal-Rausch-Verhältnis und das Grünlicht-Signal-Rausch-Verhältnis beide unter einem voreingestellten Signal-Rausch-Schwellenwert liegen, Festlegen des zweiten Bestimmungsergebnisses als Kennzeichen für ein-nicht normgerechtes Signal; oder, wenn entweder das Rotlicht-Signal-Rausch-Verhältnis oder das Grünlicht-Signal-Rausch-Verhältnis größer oder gleich dem Signal-Rausch-Schwellenwert ist, Festlegen des zweiten Bestimmungsergebnisses als normgerechtes Signalidentifikationszeichen;
Schritt 76: wenn das zweite Bestimmungsergebnis das nicht-normgerechte Signalidentifikationszeichen ist, Festlegen eines Rotlicht-Digitalsignals des ersten PPG-Signals als zweites Rotlicht-Digitalsignal und Festlegen eines Grünlicht-Digitalsignal des ersten PPG-Signals als zweites Grünlicht-Digitalsignal, wobei das erste PPG-Signal das Rotlicht-Digitalsignal und das Grünlicht-Digitalsignal umfasst.

6. Verfahren zum Erzeugen von PPG-Signalen gemäß Anspruch **1,** wobei das Verfahren ferner umfasst:
wenn das Bildqualitätserkennungsergebnis das nicht-normgerechte Signalidentifikationszeichen ist, Stoppen des kontinuierlichen Erfassungs- und
Bildaufnahmevorgangs und Erzeugen einer Hautoberflächenfehlerinformation, dann Übertragen der Hautoberflächenfehlerinformationen an das mobile Endgerät und Anzeigen der Hautoberflächenfehlerinformationen als Warninformationen durch das mobile Endgerät über die Anzeigeoberfläche;
wenn das erste Bestimmungsergebnis das nicht-normgerechte Signalidentifikationszeichen ist, Stoppen des kontinuierlichen Erfassungs- und Bildaufnahmevorgangs und des PPG-Signalerzeugungsprozesses und Erzeugen einer Signalqualitätsfehlerinformationen, dann Übertragen der
Signalqualitätsfehlerinformationen an das mobile Endgerät und Anzeigen der Signalqualitätsfehlerinformationen als Warninformationen durch das mobile Endgerät über die Anzeigeoberfläche; oder
wenn das zweite Bestimmungsergebnis das nicht-normgerechte Signalidentifikationszeichen ist, Stoppen des kontinuierlichen Erfassungs- und
Bildaufnahmevorgangs und des PPG-Signalerzeugungsprozesses und Erzeugen von Signalqualitätsfehlerinformationen, dann Übertragen der
Signalqualitätsfehlerinformationen an das mobile Endgerät und Anzeigen der Signalqualitätsfehlerinformationen als Warninformationen durch das mobile Endgerät über die Anzeigeoberfläche.

7. Vorrichtung, die einen Speicher und einen Prozessor umfasst, wobei der Speicher zum Speichern eines Programms verwendet wird und der Prozessor zum Ausführen des Verfahrens gemäß einem der Ansprüche 1 bis 6 verwendet wird.

8. Computerprogrammprodukt, das Anweisungen umfasst, die es einem Computer ermöglichen, das Verfahren gemäß einem der Ansprüche 1 bis 6 auszuführen, wenn es auf dem Computer ausgeführt wird.

9. Computerlesbares Speichermedium, das Anweisungen umfasst, die, wenn sie von einem Computer ausgeführt werden, den Computer veranlassen, das Verfahren gemäß einem der Ansprüche 1 bis 6 auszuführen.

## Revendications

1. Procédé de génération de signaux PPG, comprenant :
effectuer une opération d'acquisition et de photographie en continu sur une surface cutanée locale d'un corps vivant à l'aide d'un terminal mobile, et pendant le processus d'acquisition et de photographie en continu, effectuer une mise en cache continue de fragments vidéo sur les données vidéo obtenues par photographie avec un seuil de temps de fragment mis en cache prédéfini comme longueur de fragment afin de générer plusieurs données vidéo de fragments mis en cache (1) ;
effectuer l'extraction d'une trame d'images sur les données vidéo de fragments mis en cache afin de générer une séquence d'images de trames de fragments mis en cache, et effectuer la détection de la qualité d'image sur toutes les images de trames de fragments mis en cache dans la séquence d'images de trames de fragments mis en cache selon une plage de seuil de pixels de lumière rouge prédéfinie afin de générer un résultat de détection de la qualité d'image (2), dans lequel la séquence d'images de trames de fragments mis en cache comprend plusieurs images de trames de fragments mis en cache ;
lorsque le résultat de la détection de la qualité d'image est un identifiant d'image conforme à la norme, effectuer une extraction de signal de lumière rouge unidimensionnel sur toutes les images de trames de fragments mis en cache dans la séquence d'images de trames de fragments mis en cache selon la plage de seuil de pixels de lumière rouge afin de générer un premier signal numérique de lumière rouge, et effectuer une extraction de signal de lumière verte unidimensionnelle sur toutes les images de trames de fragments mis en cache dans la séquence d'images de trames de fragments mise en cache selon une plage de seuil de pixels de lumière verte prédéfinie afin de générer un premier signal numérique de lumière verte (3) ; et
effectuer un prétraitement de filtrage passe-bande du signal sur le premier signal numérique de lumière rouge conformément à une plage de seuil de fréquence de filtrage passe-bande prédéfinie afin de générer un deuxième signal numérique de lumière rouge, et effectuer un prétraitement de filtrage passe-bande du signal sur le premier signal numérique de lumière verte conformément à la plage de seuil de fréquence de filtrage passe-bande prédéfinie afin de générer un deuxième signal numérique de lumière verte ; effectuer une détermination de la différence de fréquence maximale sur le deuxième signal numérique de lumière rouge et le deuxième signal numérique de lumière verte afin de générer un premier résultat de détermination ; effectuer une détermination du rapport signal/bruit sur le deuxième signal numérique de lumière rouge et le deuxième signal numérique de lumière verte afin de générer un deuxième résultat de détermination lorsque le premier résultat de détermination est un identifiant de signal conforme à la norme ; et effectuer une génération de signal PPG sur le deuxième signal numérique de lumière rouge et le deuxième signal numérique de lumière verte afin de générer un premier signal PPG des données vidéo fragmentées mises en cache lorsque le deuxième résultat de détermination est l'identifiant de signal conforme à la norme (4) ;
**caractérisé en ce que** l'étape consistant, lorsque le résultat de la détection de la qualité d'image est un identifiant d'image conforme à la norme, en effectuant une extraction de signal de lumière rouge unidimensionnelle sur toutes les images de trame de fragment mis en cache dans la séquence d'images de trame de fragment mis en cache selon la plage de seuil de pixels de lumière rouge afin de générer un premier signal numérique de lumière rouge, et en effectuant une extraction de signal lumineux vert unidimensionnel sur toutes les images de trame de fragment mis en cache dans la séquence d'images de trame de fragment mis en cache selon une plage de seuil de pixels verts prédéfinie afin de générer un premier signal numérique de lumière verte, comprend spécifiquement :
Étape 51, lorsque le résultat de la détection de la qualité de l'image est l'identifiant d'image conforme à la norme, initialiser le premier signal numérique de lumière rouge à zéro, initialiser le premier signal numérique de lumière verte à zéro, initialiser un premier index à 1 et initialiser un premier nombre total au nombre total d'images de trame de fragments mis en cache dans la séquence d'images de trame de fragments mis en cache ;
Étape 52, définir une image de trame de premier index comme image de trame de fragment mise en cache, correspondant au premier index, dans la séquence d'images de trame de fragment mise en cache ;
Étape 53, collecter tous les pixels, répondant à la plage de seuil de pixels de lumière rouge, dans l'image de trame de premier index pour générer un ensemble de pixels rouges, calculer un nombre total de pixels inclus dans l'ensemble de pixels rouges pour générer un nombre total de pixels rouges, calculer la somme des valeurs de pixels de tous les pixels dans l'ensemble de pixels rouges pour générer une somme de valeurs de pixels rouges, et générer des données de canal de lumière rouge de trame du premier index en fonction d'un quotient obtenu en divisant la somme des valeurs des pixels rouges par le nombre total de pixels rouges ; et ajouter les données de canal de lumière rouge de trame du premier index au premier signal numérique de lumière rouge en tant que données de point de signal ;
Étape 54, collecter tous les pixels, répondant à la plage de seuil de pixels de lumière verte, dans l'image de trame de première index pour générer un ensemble de pixels verts, calculer un nombre total de pixels inclus dans l'ensemble de pixels verts pour générer un nombre total de pixels verts, calculer la somme des valeurs de pixels de tous les pixels dans l'ensemble de pixels verts pour générer une somme de valeurs de pixels verts, et générer les données de canal de lumière verte de trame du première index en fonction d'un quotient obtenu en divisant la somme des valeurs des pixels verts par le nombre total de pixels verts ; et ajouter les données de canal de lumière verte de trame du première index au premier signal numérique de lumière verte en tant que données de point de signal ;
Étape 55, augmenter le premier index de 1 ;
Étape 56, déterminer si le premier index est supérieur au premier nombre total ; si le premier index est inférieur ou égal au premier nombre total, exécuter l'étape 52 ; ou, si le premier index est supérieur au premier nombre total, exécuter l'étape 57 ; et
Étape 57, transférer le premier signal numérique de lumière rouge vers un processus de traitement supérieur en tant que résultat d'extraction de signal de lumière rouge unidimensionnel, et transférer le premier signal numérique de lumière verte vers le processus de traitement supérieur en tant que résultat d'extraction de signal de lumière verte unidimensionnel ;
dans lequel l'étape consistant à effectuer un prétraitement de filtrage passe-bande du signal sur le premier signal numérique de lumière rouge selon une plage de seuil de fréquence de filtrage passe-bande prédéfinie afin de générer un deuxième signal numérique de lumière rouge, et à effectuer un prétraitement de filtrage passe-bande du signal sur le premier signal numérique de lumière verte selon la plage de seuil de fréquence de filtrage passe-bande prédéfinie afin de générer un deuxième signal numérique de lumière verte comprend :
conformément à la plage de seuils de fréquence de filtrage passe-bande, effectuer un traitement de filtrage de signal numérique sur les points de signal de bruit à basse fréquence ayant une fréquence de signal inférieure à la plage de seuils de fréquence de filtrage passe-bande et les points de signal de bruit à haute fréquence ayant une fréquence de signal supérieure à la plage de seuils de fréquence de filtrage passe-bande dans le premier signal numérique de lumière rouge afin de générer le deuxième signal numérique de lumière rouge ; et
conformément à la plage de seuil de fréquence de filtrage passe-bande, effectuer un traitement de filtrage de signal numérique sur les points de signal de bruit à basse fréquence avec une fréquence de signal inférieure à la plage de seuil de fréquence de filtrage passe-bande et les points de signal de bruit à haute fréquence avec une fréquence de signal supérieure à la plage de seuil de fréquence de filtrage passe-bande dans le premier signal numérique de lumière verte afin de générer le deuxième signal numérique de lumière verte.

2. Procédé de génération de signaux PPG selon la revendication **1,** dans lequel le procédé comprend en outre :
effectuer un affichage en temps réel sur le premier signal PPG, et afficher localement l'image de la forme d'onde PPG en temps réel via une interface d'affichage (5).

3. Procédé de génération de signaux PPG selon la revendication **1,** dans lequel le procédé comprend en outre :
après l'opération d'acquisition et de photographie en continu, effectuer un assemblage vidéo de toutes les données vidéo de fragments mises en cache dans l'ordre chronologique par le terminal mobile afin de générer des données vidéo complètes de la surface de la peau, et envoyer les données vidéo de la surface complète de la peau à un serveur distant (101) ;
effectuer une extraction d'une trame d'images sur les données vidéo de la surface cutanée complète par le serveur afin de générer une séquence d'images de la trame vidéo de la surface cutanée complète (102), dans laquelle la séquence d'images de la trame vidéo de la surface cutanée complète comprend plusieurs images de trame vidéo de la surface cutanée complète ;
effectuer une extraction de signal de luminèrerouge unidimensionnel sur toutes les images d'images vidéo de la surface complète de la peau dans la séquence d'images de trame vidéo de la surface complète de la peau selon une plage de seuil de pixels de lumière rouge prédéfinie du serveur afin de générer un premier signal numérique de lumière rouge du serveur, et effectuer une extraction de signal de lumière vert unidimensionnel sur toutes les images d'images vidéo de la surface complète de la peau dans la séquence d'images de trame vidéo de la surface complète de la peau selon une plage de seuil de pixels lumineux vert prédéfinie du serveur afin de générer un premier signal numérique de lumière vert du serveur (103) ;
conformément à une plage de seuils de fréquence de filtrage passe-bande prédéfinie par le serveur, effectuer un prétraitement de filtrage passe-bande du signal sur le premier signal numérique de lumière rouge du serveur afin de générer un deuxième signal numérique de lumière rouge du serveur, et effectuer un prétraitement de filtrage passe-bande du signal sur le premier signal numérique de lumière verte du serveur afin de générer un deuxième signal numérique de lumière verte du serveur ; effectuer une détermination de la différence de fréquence maximale sur le deuxième signal numérique de lumière rouge du serveur et le deuxième signal numérique de lumière verte du serveur afin de générer un premier résultat de détermination du serveur ; lorsque le premier résultat de détermination du serveur est l'identifiant de signal conforme à la norme, effectuer une détermination du rapport signal/bruit sur le deuxième signal numérique de lumière rouge du serveur et le deuxième signal numérique de lumière verte du serveur afin de générer un deuxième résultat de détermination du serveur ; et lorsque le deuxième résultat de détermination du serveur est l'identifiant de signal conforme, à la norme, effectuer une génération de signal PPG sur le deuxième signal numérique de lumière rouge du serveur et le deuxième signal numérique de lumiere vert du serveur afin de générer un deuxième signal PPG des données vidéo de surface cutanée complète (104) ; et
effectuer une conversion de fichier de données PPG sur le deuxième signal PPG par le serveur afin de générer un fichier de données PPG de la surface cutanée complète, et enregistrer les données vidéo de la surface cutanée complète et le fichier de données PPG de la surface cutanée complète dans une base de données médicale (105).

4. Procédé de génération de signaux PPG selon la revendication **1,** dans lequel l'étape consistant à effectuer une extraction d'une trame d'images sur les données vidéo de fragments mis en cache afin de générer une séquence d'images de trames de fragments mis en cache, et à effectuer une détection de la qualité d'image sur toutes les images de trame de fragmentmis en cache dans la séquence d'images de trame de fragments mis en cache selon une plage de seuil de pixels de lumière rouge prédéfinie afin de générer un résultat de détection de la qualité d'image comprend :
l'extraction séquentielle de fragments de données sur les données vidéo de fragments mis en cache selon une longueur de données de trame de fragments mises en cache prédéfinie afin d'obtenir plusieurs fragments de données, générer des multiples images de trames de fragments mises en cache selon les multiples fragments de données extraits, et trier toutes les images de trames de fragments mises en cache afin de générer la séquence d'images de trames de fragments mises en cache ; et
effectuer séquentiellement l'extraction d'images de trames de fragments mis en cache sur la séquence d'images de trames de fragments mis en cache dans l'ordre chronologique afin de générer une image de trame de fragment mise en cache actuelle ; calculer un nombre total de pixels dans l'image de trame de fragment mise en cache actuelle afin de générer un nombre total de pixels, et calculer un nombre total de pixels ayant une valeur de pixel comprise dans la plage de seuil de pixels de lumière rouge afin de générer un nombre total de pixels de lumière rouge ; générer un paramètre de proportion rouge d'une trame actuelle en fonction d'un rapport entre le nombre total de pixels de lumière rouge et le nombre total de pixels ; effectuer une détection de la qualité d'image sur le paramètre de proportion rouge de l'image actuelle en fonction d'un seuil inférieur de proportion rouge prédéfini ;
lorsque le paramètre de proportion rouge de l'image actuelle est inférieur au seuil inférieur de proportion rouge, définir le résultat de la détection de la qualité d'image comme un identifiant d'image non conforme à la norme et arrêter l'extraction d'images de trame de fragments mis en cache effectuée sur la séquence d'images de fragments mis en cache ; ou,
lorsque le paramètre de proportion rouge de l'image actuelle est supérieur ou égal au seuil inférieur de proportion rouge, définir le résultat de la détection de la qualité d'image comme identifiant d'image conforme à la norme et continuer à effectuer l'extraction d'images de trames de fragments mis en cache sur la séquence d'images de fragments mis en cache.

5. Procédé de génération de signaux PPG selon la revendication 1, dans lequel l'étape consistant à effectuer une détermination de la différence de fréquence maximale sur le deuxième signal numérique de lumière rouge et le deuxième signal numérique de lumière verte afin de générer un premier résultat de détermination, effectuer la détermination du rapport signal/bruit sur le deuxième signal numérique de lumière rouge et le deuxième signal numérique de lumière verte afin de générer un deuxième résultat de détermination lorsque le premier résultat de détermination est un identifiant de signal conforme à la norme, et effectuer la génération de signal PPG sur le deuxième signal numérique de lumière rouge et le deuxième signal numérique de lumière verte afin de générer un premier signal PPG des données vidéo de fragment mis en cache lorsque le deuxième résultat de détermination est l'identifiant de signal conforme à la norme, comprend :
Étape 71, effectuer une conversion du domaine temporel au domaine fréquentiel du signal numérique sur le deuxième signal numérique de lumière rouge par transformation de Fourier discrète afin de générer un signal de domaine fréquentiel de lumière rouge, effectuer une conversion du domaine temporel au domaine fréquentiel du signal numérique sur le deuxième signal numérique de lumière verte par transformation de Fourier discrète afin de générer un signal de domaine fréquentiel de lumière verte ; extraire une fréquence d'énergie maximale du signal de domaine fréquentiel de lumière rouge afin de générer une fréquence maximale de lumière rouge, et extraire une fréquence d'énergie maximale du signal de domaine fréquentiel de lumière verte afin de générer une fréquence maximale de lumière verte ; calculer une différence de fréquence entre la fréquence maximale de la lumière rouge et la fréquence maximale de la lumière verte afin de générer une différence de fréquence maximale entre le rouge et le vert ; lorsque la différence de fréquence maximale entre le rouge et le vert ne dépasse pas une plage seuil de différence de fréquence maximale prédéfinie, définir le premier résultat de détermination comme identifiant de signal conforme à la norme ; ou, lorsque la différence de fréquence maximale entre le rouge et le vert dépasse la plage seuil de différence de fréquence maximale, définir le premier résultat de détermination comme identifiant de signal non conforme à la norme ;
Étape 72, lorsque le premier résultat de détermination est l'identifiant de signal conforme à la norme, conformément à une plage de seuil de fréquence de filtrage coupe-bande prédéfinie, supprimer les points de signal valides dont la fréquence de signal correspond à la plage de seuil de fréquence de filtrage coupe-bande du deuxième signal numérique d' de lumière rouge par un filtrage coupe-bande Butterworth à plusieurs ordres afin de générer un signal de bruit de lumière rouge, et supprimer les points de signal valides dont la fréquence de signal correspond à la plage de seuil de fréquence de filtrage coupe-bande du deuxième signal numérique de lumière verte par un filtrage coupe-bande Butterworth à plusieurs ordres afin de générer un signal de bruit de lumière verte ;
Étape 73, calculer l'énergie du deuxième signal numérique de lumière rouge pour générer l'énergie du signal de lumière rouge, calculer l'énergie du signal de bruit de lumière rouge pour générer l'énergie de bruit de lumière rouge, générer l'énergie valide du signal de lumière rouge en fonction de la différence entre l'énergie du signal de lumière rouge et
l'énergie de bruit de lumière rouge, et générer un rapport signal/bruit de lumière rouge en fonction du rapport entre l'énergie valide du signal de lumière rouge et l'énergie de bruit de lumière rouge ;
Étape 74, calculer l'énergie du deuxième signal numérique de lumière verte pour générer l'énergie du signal de lumière verte, calculer l'énergie du signal de bruit de lumière verte pour générer l'énergie de bruit de lumière verte, générer une énergie de signal de lumière verte valide en fonction de la différence entre l'énergie du signal de lumière verte et l'énergie de bruit de la lumière verte, et générer un rapport signal/bruit de la lumière verte en fonction du rapport entre l'énergie de signal de lumière verte valide et l'énergie de bruit de la lumière verte ;
Étape 75, lorsque le rapport signal/bruit de la lumière rouge et le rapport signal/bruit de la lumière verte sont tous deux inférieurs à un seuil signal/bruit prédéfini, définir le deuxième résultat de détermination comme l'identifiant de signal non conforme à la norme ; ou, lorsque l'un quelconque du rapport signal/bruit du lumière rouge et du rapport signal/bruit du lumière vert est supérieur ou égal au seuil signal/bruit, définir le deuxième résultat de détermination comme l'identifiant de signal conforme à la norme;
Étape 76, lorsque le deuxième résultat de détermination est l'identifiant de signal conforme à la norme, définir un signal numérique de lumière rouge du premier signal PPG comme deuxième signal numérique de lumière rouge, et définir un signal numérique de lumière verte du premier signal PPG comme deuxième signal numérique de lumière verte, dans lequel le premier signal PPG comprend le signal numérique de lumière rouge et le signal numérique de lumière verte.

6. Procédé de génération de signaux PPG selon la revendication 1, dans lequel le procédé comprend en outre :
lorsque le résultat de la détection de la qualité d'image est l'identifiant de signal non conforme à la forme, arrêterl'opération d'acquisition et de photographie en continu et
générer d'informations d'erreur de surface hors de la peau, puis transférer les informations d'erreur de surface hors de la peau vers le terminal mobile, et afficher, par le terminal mobile, des informations d'erreur de surface hors de la peau comme informations d'avertissement via l'interface d'affichage ;
lorsque le premier résultat de détermination est l'identifiant de signal non conforme à la norme, arrêter l'opération d'acquisition et de photographie en continu et le processus de génération de signaux PPG, générer d'informations d'erreur de qualité du signal, puis transfére les informations d'erreur de qualité du signal vers le terminal mobile, et afficher, par le terminal mobile, des informations d'erreur de qualité du signal comme informations d'avertissement via l'interface d'affichage ; ou
lorsque le deuxième résultat de détermination est l'identifiant de signal non conforme à la norme, arrêter l'opération d'acquisition et de photographie en continu et le processus de génération de signal PPG et générer des informations d'erreur de qualité du signal, puis transférer les informations d'erreur de qualité du signal vers le terminal mobile, et afficher, par le terminal mobile, les informations d'erreur de qualité du signal comme informations d'avertissement via l'interface d'affichage.

7. Équipement comprenant une mémoire et un processeur, dans lequel la mémoire est utilisée pour stocker un programme, et le processeur est utilisé pour mettre en œuvre le procédé selon l'une quelconque des revendications 1 à 6.

8. Produit logiciel comprenant des instructions permettant à un ordinateur de mettre en œuvre le procédé selon l'une quelconque des revendications 1 à 6 lorsqu'il est exécuté sur l'ordinateur.

9. Support de stockage lisible par ordinateur comprenant des instructions qui, lorsqu'elles sont exécutées par un ordinateur, amènent l'ordinateur à mettre en œuvre le procédé selon l'une quelconque des revendications 1 à 6.
